# EUROPEAN PATENT APPLICATION

(11) **EP 1 683 796 A1**
(43) Date of publication of application: **26.07.2006**
(21) Application number: 05075177.5
(22) Date of filing: 24.01.2005
(51) Int. Cl.: C07D 471/04, A61K 31/437, A61P 35/00, A61P 29/00, A61P 27/00, A61P 17/00

(54) **Pyrazolopyridines, their preparation and their medical use**

(71) Applicant: Schering Aktiengesellschaft, 13353 Berlin (DE)
(72) Inventor: Schwede, Wolfgang, 16548 Glienicke (DE); Briem, Hans, 28279 Bremen (DE); Künzer, Hermann, 13348 Berlin (DE); Husemann, Manfred, 16540 Hohen Neuendorf (DE); Kettschau, Georg, 13187 Berlin (DE); Schäfer, Martina, 13187 Berlin (DE); Ter Laak, Antonius, 12159 Berlin (DE); Thierauch, Karl-Heinz, 14169 Berlin (DE); Ince, Stuart James, 10559 Berlin (DE)

(57) **Abstract**

The invention relates to pyrazolopyridines according to the general Formula I 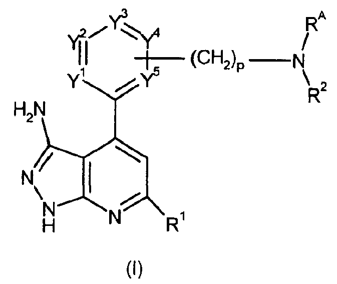 and the salts thereof, to pharmaceutical compositions comprising the pyrazolopyridines and to a method of preparing the pyrazolopyrimidines as well as the use thereof for manufacturing a pharmaceutical composition for the treatment of diseases of dysregulated vascular growth or of diseases which are accompanied with dysregulated vascular growth, wherein the compounds effectively interfere with angiopoietin and therefore influence Tie2 signalling.

## Description

The invention relates to pyrazolopyridine compounds of general formula (I) and the salts thereof, to pharmaceutical compositions comprising the pyrazolopyrimidine compounds, to methods of preparing the sulfonamido-macrocycles as well as to the use thereof.

In order to defeat diseases with dysregulated vascular growth like cancer different strategies were developed. One possible strategy is the blockade of angiogenesis to the tumour tissue, because tumour angiogenesis is a prerequisite for the growth of solid tumours.

The angiogenesis represents beside the vasculogenesis one of two basic processes during the genesis of vasculature. Vasculogensis names the neoplasm of vascular tissue during the embryo development, wherein the angiogenesis describes the neoplasm of vasculature by sprouts or division of present vasculature. It has been found that two receptors expressed on endothelial cells, VEGF- (vascular endothelial growth factor) and Tie-receptors, are essential for normal development of vasculature as blood vessels (Dumont *et al.,* (1994). Dominant-negative and targeted null mutations in the endothelial receptor tyrosine kinase, tek, reveal a critical role in vasculogenesis of the embryo. Genes *Dev,* **8:**1897-909; Sato *et al.:* "Distinct roles of the receptor tyrosine kinases Tie-1 and Tie-2 in blood vessel formation" *Nature.* **1995,** Jul 6; 376(6535):70-4.).

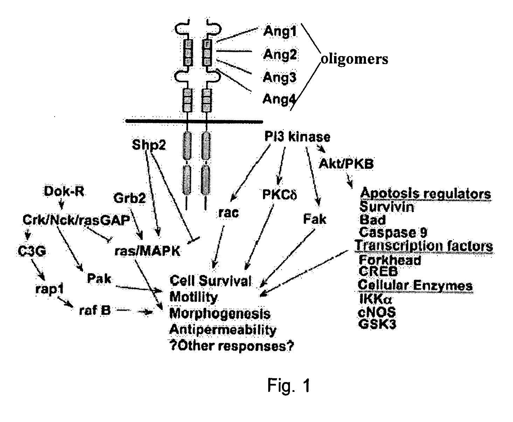

The mechanism of Tie2 signalling was characterized by different searchers, wherein different angiopoietins were found to be involved. So it could be explained that angiopoietin-1 if bound to the extracellular domain of the Tie2-receptor stimulates autophosphorylation and activates the intracellular kinase domain. Angiopoietin-1 activation of Tie2, however, does not stimulate mitogenesis but rather migration. Angiopoietin-2 can block angiopoietin-1 mediated Tie2 activation and the resulting endothelial migration. This indicates that angiopoietin-2 is a naturally occurring inhibitor of Tie2 activation (Maisonpierre *et al.:* "Angiopoietin-2, a natural antagonist for Tie2 that disrupts in vivo angiogenesis". Science. 1997, Jul 4; 277(5322):55-60; Witzenbichler *et al*.: "Chemotactic properties of angiopoietin-1 and -2, ligands for the endothelial-specific receptor tyrosine kinase Tie2". *J Biol Chem.* **1998,** Jul 17; 273(29):18514-21). For an overview see Figure 1 modified by Peters *et al.* (Peters *et al.:* "Functional significance of Tie2 signalling in the adult vasculature". *Recent Prog Horm Res.* **2004;** 59:51-71. Review.).

Receptor dimerization results in cross-phosphorylation on specific tyrosine-residues. Receptor cross-phosphorylation has a dual effect: it enhances the receptor's kinase activity and it provides binding sites for signalling molecules possessing phosphotyrosine binding domains (SH2 and PTB domains) (Pawson T.: "Regulation and targets of receptor tyrosine kinases". *Eur J Cancer.* **2002,** Sep, 38 Suppl 5:S3-10. Review).

The signalling cross-talk between the P13-K pathway and the Dok-R pathway is required for an optimal chemotactic response downstream of Tie2. Other recent studies have shown that Tie2-mediated activation of the P13-K/Akt pathway is required for endothelial nitric oxide synthase (eNOS) activation, focal adhesion kinase activation, and protease secretion, all of which may contribute importantly to Tie2 function during angiogenesis (Kim I. *et al.:* "Angiopoietin-1 regulates endothelial cell survival through the phosphatidylinositol 3'-Kinase/Akt signal transduction pathway". *Circ Res.* **2000,** Jan 7-21; 86(1):24-9; Babaei *et al*.: "Angiogenic actions of angiopoietin-1 require endothelium-derived nitric oxide". *Am J Pathol.* **2003,** Jun; 162(6):1927-36).

For normal development a balanced interaction between the receptors and so-called ligands is necessary. Especially the angiopoietins, which signal via Tie2 receptors, play an important role in angiogenesis (Babaei *et al.,* **2003).**

The broad expression of Tie2 in adult vasculature has been confirmed in transgenic mice using Tie2 promoter driven reporters (Schlaeger *et al.:* "Uniform vascular-endothelial-cell-specific gene expression in both embryonic and adult transgenic mice". *Proc Natl Acad Sci U S A.* **1997,** Apr 1; 94(7):3058-63; Motoike *et al.:* "Universal GFP reporter for the study of vascular development". Genesis. **2000,** Oct; 28(2):75-81). Immunohistochemical analysis demonstrated the expression of Tie2 in adult rat tissues undergoing angiogenesis. During ovarian folliculogenesis, Tie2 was expressed in the neo-vessels of the developing corpus luteum. Angiopoietin-1 and angiopoietin-2 also were expressed in the corpus luteum, with angiopoietin-2 localizing to the leading edge of proliferating vessels and angiopoietin-1 localizing diffusely behind the leading edge (Maisonpierre *et al.,* 1997). It was suggested that angiopoietin-2-mediated inhibition of Tie2 activation serves to "destabilize" the vessel, to make it responsive to other angiogenic growth factors such as VEGF. Subsequently, angiopoietin-1-mediated activation of Tie2 would trigger stabilization of the neovasculature.

The disruption of Tie2 function shows the relevance of Tie2 for neoangiogenesis in transgenic mice resulting in early embryonic lethality as a consequence of vascular abnormalities (Dumont *et al.,* **1994;** Sato *et al.,* **1995).** Tie2-/- embryos failed to develop the normal vessel hierarchy, suggestive of a failure of vascular branching and differentiation. Tie2-/- embryos have a decreased number of endothelial cells and furthermore less contact between endothelial cells and the underlying pericytes/smooth muscle cells. This implies a role in the maturation and stabilization of newly formed vasculature.

The studies in mice with transgenic or ablated Tie2 gene suggest a critical role for Tie2 in maturation of vascular development in embryos and in adult vasculature. Conditional expression of Tie2 in the endothelium of mice homozygous for a Tie2 null allele partially rescued the embryonic lethality of the Tie2 null phenotype (Jones *et al.,* "Tie receptors: new modulators of angiogenic and lymphangiogenic responses", Nat Rev Mol Cell Biol. **2001** Apr;2(4):257-67. Review). Mice lacking functional angiopoietin-1 expression and mice overexpressing angiopoietin-2 both displayed a phenotype similar to Tie2-/mice (Suri *et al.,* "Requisite role of angiopoietin-1, a ligand for the TIE2 receptor, during embryonic angiogenesis.", Cell. **1996** Dec 27;87(7):1171-80; Maisonpierre *et al.,* "Angiopoietin-2, a natural antagonist for Tie2 that disrupts in vivo angiogenesis.", Science, **1997** Jul 4;277(5322):55-60).

Angiopoietin-2 -/- mice have profound defects in the growth and patterning of lymphatic vasculature and fail to remodel and regress the hyaloid vasculature of the neonatal lens (Gale *et al.:* "Angiopoietin 2 is required for postnatal angiogenesis and lymphatic patterning, and only the latter role is rescued by Angiopoietin-1". *Dev Cell.* **2002,** Sep; 3(3):411-23). Angiopoietin-1 rescued the lymphatic defects, but not the vascular remodeling defects. So angiopoietin-2 might function as a Tie2 antagonist in blood vasculature but as a Tie2 agonist in developing lymph vasculature.

Tie2 also plays a role in pathological angiogenesis. It was shown that mutations in Tie2 that cause inherited venous malformations and enhance both ligand dependent and independent Tie2 kinase activity (Vikkula *et al.:* "Dysmorphogenesis caused by an activating mutation in the receptor tyrosine kinase TIE2". Cell. **1996,** Dec 27; 87(7):1181-90). Tie2 expression was investigated in human breast cancer tumour specimens and Tie2 expression was found in the vascular endothelium both in normal breast tissue and in breast tumours. The proportion of Tie2-positive tumour microvessels was increased in tumours as compared to normal breast tissue (Peters *et al.,* "Expression of Tie2/Tek in breast tumour vasculature provides a new marker for evaluation of tumour angiogenesis.", Br J Cancer. **1998** ;77(1):51-6).

Angiopoietin-1 overexpression in tumour models resulted in decreased tumour growth. The effect is possibly related to angiopoietin-1 mediated stabilization of the tumour vasculature, which renders the vessels resistant to angiogenic stimuli (Hayes *et al.:* "Expression and function of angiopoietin-1 in breast cancer". *BrJ Cancer.* **2000,** Nov; 83(9):1154-60; Shim *et al.:* 'inhibition of angiopoietin-1 expression in tumour cells by an antisense RNA approach inhibited xenograft tumour growth in immunodeficient mice". *Int J Cancer.* **2001,** Oct 1; 94(1):6-15; Shim *et al.:* "Angiopoietin 1 promotes tumour angiogenesis and tumour vessel plasticity of human cervical cancer in mice". *Exp Cell Res.* **2002,** Oct 1; 279(2):299-309; Hawighorst *et al.:* "Activation of the tie2 receptor by angiopoietin-1 enhances tumour vessel maturation and impairs squamous cell carcinoma growth". *Am J Pathol.* **2002,** Apr;160(4):1381-92.; Stoeltzing et al.: "Angiopoietin-1 inhibits vascular permeability, angiogenesis, and growth of hepatic colon cancer tumours". *Cancer Res.* **2003,** Jun 15; 63(12):3370-7.).

Corneal angiogenesis induced by tumour cell conditioned medium was inhibited by recombinant sTie, despite the presence of VEGF. Mammary tumour growth was significantly inhibited in a skin chamber tumour model recombinant sTie2 (Lin *et al.:* "Inhibition of tumour angiogenesis using a soluble receptor establishes a role for Tie2 in pathologic vascular growth". *J Clin Invest.* **1997,** Oct 15;100(8):2072-8; Lin *et al.:* "Antiangiogenic gene therapy targeting the endothelium-specific receptor tyrosine kinase Tie2". *Proc Natl Acad Sci U S A.* **1998,** Jul 21; 95(15):8829-34). Similar sTie constructs have shown comparable effects in different tumour models (Siemeister *et al.:* "Two independent mechanisms essential for tumor angiogenesis: inhibition of human melanoma xenograft growth by interfering with either the vascular endothelial growth factor receptor pathway or the Tie-2 pathway". *Cancer Res.* **1999,** Jul 1; 59(13):3185-91; Stratmann *et al.:* "Differential inhibition of tumor angiogenesis by tie2 and vascular endothelial growth factor receptor-2 dominant-negative receptor mutants". *Int J Cancer.* **2001,** Feb 1; 91(3):273-82; Tanaka *et al.:* "Tie2 vascular endothelial receptor expression and function in hepatocellular carcinoma". *Hepatology.* **2002,** Apr; 35(4):861-7).

When the interaction of angiopoietin-2 with its receptor is blocked by application of a neutralizing anti-angiopoietin-2 monoclonal antibody, the growth of experimental tumours can be blocked efficiently again pointing to the important role of Tie2 in tumour angiogenesis and growth (Oliner *et al.:* "Suppression of angiogenesis and tumor growth by selective inhibition of angiopoietin-2". *Cancer Cell.* **2004,** Nov; 6(5):507-16.) So inhibiting the Tie2 pathway will inhibit pathological angiogenesis.

To influence the interaction between receptor and ligand it could be shown that angiogenesis may be blocked with blockers like Avastin which interfere with VEGF signal transduction to endothelial cells.

Avastin is a clinically effective antibody that functions as tumour growth inhibitor by blockade of VEGFR mediated angiogenic signalling. Thus interference with VEGF signalling is a proven clinical principle. VEGF-C is a molecule inducing lymph angiogenesis via VEGFR 3. The blockade of this signal pathway is inhibiting diseases associated with lymph angiogenesis as is lymphedema and related diseases (Saharinen *et al.:* "Lymphatic vasculature: development, molecular regulation and role in tumour metastasis and inflammation." *Trends Immunol.* **2004,** Jul:25(7): 387-95. Review).

Pyrazolopyridine have been disclosed as antimicrobiotic substances (e.g. Attaby et al, Phosphorus, Sulfur and Silicon and the related Elements (1999), 149, 49-64; ibid. (1999), 155, 253-270).

US 5,478,830 further discloses fused heterocycles for the treatment of atherisclerosis.

WO 01/19828 (BASF AG) discloses 125 templates, including pyrazolopyrimidine as kinase inhibitors.

However, the state of the art does not disclose any pyrazolopyridines as Tie2 inhibitors.

Since there is a high demand for active compounds which can be used as inhibitors of Tie2 and in the treatment of for the treatment of diseases of dysregulated vascular growth or diseases which are accompanied with dysregulated vascular growth, in particular solid tumors and metastases thereof, it is the aim of the present invention to provide novel compounds, which inhibit Tie 2 and are useful for the treatment of diseases of dysregulated vascular growth or diseases which are accompanied with dysregulated vascular growth, in particular solid tumors and metastases thereof.

The solution to the above problems is achieved by providing compounds derived from a class of pyrazolopyridines and the salts thereof in accordance with claim 1, methods of preparing pyrazolopyrimidines in accordance with claims 5 and 6, a pharmaceutical composition in accordance with claim 7, the use of the compounds in accordance with claim 8 and a method for treating diseases with the inventive compounds in accordance with claim 15.

The invention thus relates to compounds of general formula (I), 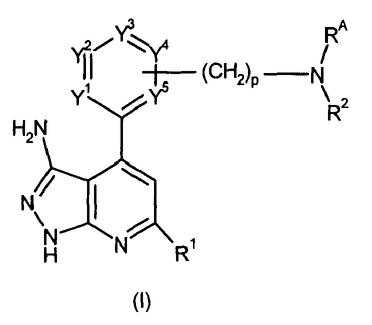 wherein
- R¹: stands for C₁-C₆-alkyl, C₂-C₆-alkenyl, C₂-C₆-alkinyl, C₃-C₁₀₋cycloalkyl, C₃-C₁ₒ-heterocycloalkyl, aryl or heteroaryl optionally being substituted one or more times, the same way or differently with K, whereby C₃-C₁₀-heterocycloalkyl itself must at least once be interrupted by an atom of the group comprising nitrogen, oxygen and/or sulfur and whereby C₃-C₁₀-cydoalkyl ring and/or C₃-C₁₀₋heterocycloalkyl ring can optionally be interrupted one or more times, the same or differently with a group -(CO)-, -SO- or -SO₂- and whereby C₃-C₁₀-cycloalkyl ring and/or C₃-C₁₀-heterocycloalkyl ring can optionally contain one or more double bonds,
- K: stands for halogen, hydroxy or a substituent of the group comprising -O-R³ or -NR⁵R⁶
or for C₃-C₁₀-cycloalkyl, C₃-C₁₀-heterocycloalkyl, aryl or heteroaryl optionally being substituted one or more times, the same way or differently with L, whereby C₃-C₁₀-heterocycloalkyl itself must at least one time be interrupted by an atom of the group comprising nitrogen, oxygen and/or sulfur and whereby C₃-C₁₀-cycloalkyl ring and/or C₃-C₁₀-heterocycloalkyl ring can optionally be interrupted one or more times, the same or differently with a group -(CO)-, -SO- or -SO₂- and whereby C₃-C₁₀-cycloalkyl ring and/or C₃-C₁₀₋heterocycloalkyl ring can optionally contain one or more double bonds,
- L: stands for a substituent of the group comprising -COR⁴ or -NR⁵R⁶ or for C₁-C₆-alkyl optionally being substituted one or more times, the same way or differently with halogen, hydroxy, cyano, nitro or the group -NR⁵R⁶ or -COR⁴,
- R^{A}: stands for hydrogen or C₁-C₆-alkyl optionally being substituted one or more times, the same way or differently with halogen, hydroxy, cyano, nitro or the group - NR⁵R⁶ or -COR⁴,
- R²: stands for a substituent of the group comprising -C(O)-NR⁷R^{7a}, -S(O)₂-R⁷, -S(O)₂NR⁷R^{7a}, -S(O)(NH)R⁷, -C(O)R⁷ or -C(O)OR⁷,
- R³: stands for C₁-C₆-alkyl, aryl or -(CH₂)ₙ-aryl, wherein C₁-C₆-alkyl, aryl or -(CH₂)ₙ-aryl can optionally be substituted one or more times, the same way or differently with C₁-C₆-alkyl, C₁-C₆-alkoxy, halogen, hydroxy, cyano, nitro or the group - NR⁵R⁶ or -COR⁴,
- R⁴: stands for hydrogen, hydroxy, C₁-C₆-alkyl, C₁-C₆-alkoxy or the group -NR⁵R⁶,
- R⁵ and R⁶: independently from another stand for hydrogen, C₁-C₆-alkyl, aryl or for a group -COR⁴ , wherein C₁-C₆-alkyl or aryl can optionally be substituted one or more times, the same way or differently with halogen, hydroxy, cyano, nitro or the group - NR⁷R^{7a} or -COR⁴,
or
- R⁵ and R⁶: together with nitrogen form a 3 to 10 membered heterocycloalkyl ring, whereby heterocycloalkyl ring itself must at least one time be interrupted by an atom of the group comprising nitrogen, oxygen and/or sulfur and can optionally be interrupted one or more times, the same way or differently with a group -(CO)-, -SO- and/or -SO₂- and can optionally contain one or more double bonds,
- R⁷ and R^{7a}: independently from another stand for hydrogen, C₁-C₆-alkyl , C₃-C₁₀₋cycloalkyl, C₃-C₁₀-heterocycloalkyl, aryl or heteroaryl, wherein C₁₋C₆-alkyl , C₃-C₁₀-cycloalkyl, C₃-C₁₀-heterocycloalkyl, aryl or heteroaryl can optionally be substituted one or more times, the same way or differently with M, whereby C₃-C₁₀-heterocycloalkyl itself must at least one time be interrupted by an atom of the group comprising nitrogen, oxygen and/or sulfur and whereby C₃-C₁₀₋cycloalkylring and/or C₃-C₁₀-heterocycloalkyl ring can optionally be interrupted one or more times, the same way or differently with a group -(CO)-, -SO- or -SO₂- and whereby C₃-C₁₀-cycloalkyl ring and/or C₃-C₁₀-heterocycloalkyl ring can optionally contain one or more double bonds,
or
- R⁷ and R^{7a}: together with nitrogen form a 3 to 10 membered heterocycloalkyl ring, whereby heterocycloalkyl ring itself must at least one time be interrupted by an atom of the group comprising nitrogen, oxygen and/or sulfur and can optionally be interrupted one or more times, the same way or differently with a group -(CO)-, -SO- or -SO₂- and can optionally contain one or more double bonds,
- M: stands for a substituent of the group comprising cyano, halogen, hydroxy, nitro or for C₁-C₆-alkyl, C₂-C₆-alkenyl, C₂-C₆-alkinyl, C₁-C₆₋alkoxy, aryl, C₃-C₁₀-heteroaryl, -(CH₂)ₙ-phenyl or -(CH₂)ₚ-O-(CH₂)ₚphenyl, wherein C₁-C₆-alkyl, C₂-C₆-alkenyl, C₂-C₆-alkinyl, C₁₋C₆-alkoxy, aryl, C₃-C₁₀-heteroaryl, -(CH₂)ₙ-phenyl or -(CH₂)ₚ-O-(CH₂)ₚphenyl can optionally be substituted one or more times, the same way or differently with a substituent of the group comprising amino, cyano, halogen, hydroxy, nitro, C₁-C₆-alkoxy, -(CH₂)ₚ-phenyl or -(CH₂)ₚ-O-(CH₂)ₚphenyl,
- Y¹, Y², Y³, Y⁴ and Y⁵: independently from another stand for -CH=, -CZ= or -N= and -N= can stand from 0 to 3 times as a ringatom,
- Z: stands for cyano, nitro, halogen, hydroxy or a substituent of the group comprising -NR⁵R⁶, -OR³, -C(O)R⁷, -C(O)OR⁷, -C(O)NR⁷R^{7a} , -S(O)R⁷ or -S(O)₂R⁷ or for C₁-C₆-alkyl, C₂-C₆-alkenyl, C₂-C₆-alkinyl or C₃-C₁₀-cycloalkyl, whereby C₁-C₆-alkyl, C₂-C₆-alkenyl, C₂-C₆₋alkinyl or C₃-C₁₀-cycloalkyl can optionally be substituted one or more times, the same way or different with cyano, nitro, halogen, hydroxy, -OR³ or -NR⁵R⁶,
- n: stands for 1 to 4 and
- p: stands for 0 to 4 as well as
N-oxides, solvates, hydrates, isomers, diastereomers, enantiomers and salts thereof, which are effective inhibitors of Tie2 kinase.

The terms as mentioned herein below and in the claims have preferably the following meanings:
The term alkyl preferably refers to branched and unbranched alkyl, meaning *e.g*. methyl, ethyl, n-propyl, *iso-*propyl, *n*-butyl, *iso*-butyl, *tert*-butyl, *sec*-butyl, pentyl, *iso*-pentyl, hexyl, heptyl, octyl, nonyl and decyl and the isomers thereof.
The term alkoxy preferably refers to branched and unbranched alkoxy, meaning *e.g*. methoxy, ethoxy, propyloxy, *iso-*propyloxy, butyloxy, *iso*-butyloxy, *tert* butyloxy, *sec*-butyloxy, pentyloxy, *iso-*pentyloxy, hexyloxy, heptyloxy, octyloxy, nonyloxy, decyloxy, undecyloxy and dodecyloxy and the isomers thereof.
The term C₃-C₁₀-cycloalkyl preferably refers to cycloalkyl, meaning e.g. cyclopropyl, cyclobutyl, cyclopentyl, cyclohexyl, cycloheptyl, cyclooctyl, cyclononyl and cyclodecyl. C₃-C₁₀-cycloalkylring can optionally be interrupted one or more times, the same or differently with a group -(CO)-, -SO- or -SO₂₋and can optionally contain one or more double bonds e.g. cycloalkenyl, such as cyclopropenyl, cyclobutenyl, cyclopentenyl, cyclohexenyl and cycloheptenyl, cyclooctenyl, cyclononenyl, cyclodecenyl, wherein the linkage can be provided to the double or single bond.
The term C₃-C₁₀-heterocycloalkyl preferably is at least once interrupted by an atom, the same or different, of the group comprising nitrogen, oxygen and/or sulfur *e.g*. oxyranyl, oxetanyl, aziridinyl, azetidinyl, tetrahydrofuranyl, pyrrolidinyl, morpholinyl, dithianyl, thiomorpholinyl, piperazinyl, trithianyl and chinuclidinyl. C₃-C₁₀-heterocycloalkylring can optionally be interrupted one or more times, the same or differently with a group -(CO)-, -SO- or -SO₂- and C₃-C₁₀₋heterocycloalkylring can optionally contain one or more double bonds, e.g. 4H-Pyran, 2H-Pyran, 3H-Diazirine, 2,5-Dihydro-1H-pyrrole, [1,3]Dioxole; 4H-[1,3,4]Thiadiazine, 2,5-Dihydrofuran, 2,3-Dihydrofuran, 2,5-Dihydrothiophene, 2,3-Dihydrothiophene, 4,5-Dihydrooxazole or 4H-[1,4]Thiazine.
Halogen or Hal preferably refers to fluorine, chlorine, bromine and iodine.
The term alkenyl preferably refers to branched and unbranched alkenyl, *e.g*. to vinyl, propen-1-yl, propen-2-yl, but-1-en-1-yl, but-1-en-2-yl, but-2-en-1-yl, but-2-en-2-yl, but-1-en-3-yl, 2-methyl-prop-2-en-1-yl and 2-methyl-prop-1-en-1-yl.
The term alkinyl preferably refers to branched and unbranched alkinyl, e.g. to ethinyl, prop-1-in-1-yl, but-1-in-1-yl, but-2-in-1-yl and but-3-in-1-yl.
The term aryl refers to cyclic or polycyclic aromates having 6 to 12 carbon atoms, *e.g.* to phenyl, naphthyl and biphenyl.
The term heteroaryl refers to cyclic or polycyclic aromates having 5 to 12 ring members, *e.g*. to five-membered heteroaromates, such as thiophenyl, furanyl, oxazolyl, thiazolyl, imidazolyl, pyrazolyl, triazolyl, thia-4H-pyrazolyl and benzo-derivates thereof, or six-membered heteroaromates, such as pyridinyl, pyrimidinyl, triazinyl, and benzo-derivates thereof, or 12 membered heteroaromates, such as quinolinyl, isoquinolinyl *etc.* .
The term isomers refers to chemical compounds with the same number and types of atoms as another chemical species. There are two main classes of isomers, constitutional isomers and stereoisomers.
The term constitutional isomers refers to chemical compounds with the same number and types of atoms, but they are connected in differing sequences. There are functional isomers, structural isomers, tautomers or valence isomers.

In stereoisomers, the atoms are connected sequentially in the same way, such that condensed formulae for two isomeric molecules are identical. The isomers differ, however, in the way the atoms are arranged in space. There are two major sub-classes of stereoisomers; conformational isomers, which interconvert through rotations around single bonds, and configurational isomers, which are not readily interconvertable.

Configurational isomers are, in turn, comprised of enantiomers and diastereomers. Enantiomers are stereoisomers which are related to each other as mirror images. Enantiomers can contain any number of stereogenic centers, as long as each center is the exact mirror image of the corresponding center in the other molecule. If one or more of these centers differs in configuration, the two molecules are no longer mirror images. Stereoisomers which are not enantiomers are called diastereomers. Diastereomers which still have a different constitution, are another sub-class of diastereomers, the best known of which are simple *cis - trans* isomers.

In order to limit different types of isomers from each other reference is made to IUPAC Roles Section E *(Pure Appl Chem* **45**, 11-30, 1976).

The compound according to Fomula **I** (pyrazolopyridine) can exist in free form or in a salt form. A suitably pharmaceutically acceptable salt of the sulfonamido-macrocycles of the invention is, for example, an acid-addition salt of a sulfonamido-macrocycle of the invention which is sufficiently basic, for example, an acid-addition salt with, for example, an inorganic or organic acid, for example hydrochloric, hydrobromic, sulphuric, phosphoric, trifluoroacetic, citric or maleic acid. In addition a suitably pharmaceutically acceptable salt of a sulfonamido-macrocycle of the invention which is sufficiently acidic is an alkali metal salt, for example a sodium or potassium salt, an alkaline earth metal salt, for example a calcium or magnesium salt, an ammonium salt or a salt with an organic base which affords a physiologically acceptable cation, for example a salt with N-methyl-glukamine, dimethyl-glukamine, ethyl-glukamine, lysine, 1,6-hexadiamin, ethanolamine, glucosamine, sarkosine, serinole, tris-hydroxy-methyl-aminomethan, aminopropandiole, sovak-base, 1-amino-2,3,4-butantriole.

The compound according to Formula I (pyrazolopyridine) can exist as N-oxides which are defined in that at least one nitrogen of the compounds of the general Formula I may be oxidized.

The compound according to Formula I (pyrazolopyridine) can exist as solvates, in particular as hydrate, wherein the compound according to Formula I may contain polar solvents, in particular water, as structural element of the crystal lattice of the compounds. The amount of polar solvents, in particular water, may exist in a stoichiometric or unstoichiometric ratio. In case of stoichiometric solvates, e.g. hydrate, are possible hemi-, (semi-), mono-, sesqui-, di-, tri-, tetra-, penta- etc. solvates or hydrates, respectively.

Compounds of general formula (I) are particularly preferred, wherein
- R¹: stands for C₁-C₆-alkyl, C₂-C₆-alkenyl, C₂-C₆-alkinyl, C₃-C₁₀₋cycloalkyl, aryl or heteroaryl optionally being substituted one or more times, the same way or differently with K,
- K: stands for halogen, hydroxy or stands for morpholinyl, piperazinyl, piperidinyl, pyrrolidinyl or phenoxy optionally substituted with L,
- L: stands for C₁-C₆-alkyl or -COO-C₁-C₆ alkyl, whereby C₁-C₆-alkyl or -COO-C₁-C₆ alkyl can optionally be substituted one or more times, the same way or differently with halogen,
- R^{A}: stands for hydrogen or C₁-C₆-alkyl optionally being substituted one or more times, the same way or differently with halogen, hydroxy, cyano, nitro or the group - NR⁵R⁶ or -COR⁴,
- R²: stands for a substituent of the group comprising -C(O)-NR⁷R^{7a}, -S(O)₂-R⁷, -S(O)₂NR⁷R^{7a}, -S(O)(NH)R⁷, -C(O)R⁷ or -C(O)OR⁷,
- R³: stands for C₁-C₆-alkyl, aryl or -(CH₂)ₙ-aryl, wherein C₁-C₆-alkyl, aryl or -(CH₂)ₙ-aryl can optionally be substituted one or more times, the same way or differently with C₁-C₆-alkyl, C₁-C₆-alkoxy, halogen, hydroxy, cyano, nitro or the group - NR⁵R⁶ or -COR⁴,
- R⁵ and R⁶: independently from another stand for hydrogen, C₁-C₆-alkyl, aryl or for a group -COR⁴, wherein C₁-C₆-alkyl or aryl can optionally be substituted one or more times, the same way or differently with halogen, hydroxy, cyano, nitro or the group - NR⁷R^{7a} or -COR⁴,
- R⁷ and R^{7a}: independently from another stand for hydrogen, aryl or heteroaryl optionally being substituted one or more times, the same way or differently with M,
- M: stands for a substituent of the group comprising cyano, halogen, hydroxy, nitro or for C₁-C₆-alkyl, C₂-C₆-alkenyl, C₂-C₆-alkinyl, C₁-C₆₋alkoxy, aryl, C₃-C₁₀-heteroaryl, -(CH₂)ₙ-phenyl or -(CH₂)ₚ-O-(CH₂)ₚphenyl, wherein C₁-C₆-alkyl, C₂-C₆-alkenyl, C₂-C₆-alkinyl, C₁₋C₆-alkoxy, aryl, C₃-C₁₀-heteroaryl, -(CH₂)ₙ-phenyl) or -(CH₂)ₚ-O-(CH₂)ₚphenyl can optionally be substituted one or more times, the same way or differently with a substituent of the group comprising amino, cyano, halogen, hydroxy, nitro or C₁-C₆-alkoxy,
- Y¹ , Y², Y³, Y⁴ and Y⁵: independently from another stand for -CH=, -CZ= or -N= and -N= can stand from 0 to 3 times as a ringatom,
- Z: stands for cyano, nitro, halogen, hydroxy or a substituent of the group comprising -NR⁵R⁶, -OR³, -C(O)R⁷, -C(O)OR⁷, -C(O)NR⁷R^{7a} -S(O)R⁷ or -S(O)₂R⁷ or for C₁-C₆-alkyl, C₂-C₆-alkenyl, C₂-C₆-alkinyl or C₃-C₁₀-cycloalkyl, whereby C₁-C₆-alkyl, C₂-C₆-alkenyl, C₂-C₆₋alkinyl or C₃-C₁₀-cycloalkyl can optionally be substituted one or more times, the same way or different with cyano, nitro, halogen, hydroxy, -OR³ or -NR⁵R⁶,
- n: stands for 1 to 4 and
- p: stands for 0 to 4
as well as N-oxides, solvates, hydrates, isomers, diastereomers, enantiomers and salts thereof.

Further preferred are compounds of general formula (I), wherein
- R¹: stands for C₁-C₆-alkyl or C₃-C₁₀-cycloalkyl optionally being substituted one or more times, the same way or differently with K,
- K: stands for halogen, hydroxy or stands for morpholinylene, piperazinylene, piperidinylene or phenyleneoxy,
- R^{A}: stands for hydrogen or C₁-C₆-alkyl,
- R²: stands for a substituent of the group comprising -C(O)-NH-R⁷ or -S(O)₂-R⁷,
- R⁷: stands for phenylene or naphtylene optionally being substituted one or more times, the same way or differently with M,
- M: stands for a substituent of the group comprising cyano, halogen, nitro or for C₁-C₆-alkyl optionally being substituted one or more times, the same way or differently with a substituent of the group comprising amino, cyano, halogen, hydroxy, nitro or C₁-C₆-alkoxy,
- Y¹, Y², Y³ Y⁴ and Y⁵: stand for -CH= and
- p: stands for 0 to 4
as well as N-oxides, solvates, hydrates, isomers, diastereomers, enantiomers and salts thereof.

More preferred are compounds of general formula (I), wherein
- R¹: stands for tert.-butyl or cyclopropyl,
- R^{A}: stands for hydrogen,
- R²: stands for a substituent of the group comprising -C(O)-NH-R⁷ or -S(O)₂-R⁷,
- R⁷: stands for phenylene or naphtylene optionally being substituted one or more times, the same way or differently with M,
- M: stands for a substituent of the group comprising cyano, halogen, nitro or for methyl,
- Y¹, Y², Y³ Y⁴ and Y⁵: stand for -CH= and
- p: stands for 0
as well as N-oxides, solvates, hydrates, isomers, diastereomers, enantiomers and salts thereof.

The following compounds are mostly preferred:
Example 1:
   1-[4-(3-Amino-6-tert-butyl-1 H-pyrazolo[3,4-b]pyridin-4-yl)-phenyl]-3-phenyl-urea
Example 2:
   N-[4-(3-Amino-6-tert-butyl-1 H-pyrazolo[3,4-b]pyridin-4-yl)-phenyl]-benzenesulfonamide
Example 3:
   1-[4-(3-Amino-6-tert-butyl-1H-pyrazolo[3,4-b]pyridin-4-yl)-phenyl]-3-(3-fluorophenyl)-urea
Example 4:
   1-[4-(3-Amino-6-tert-butyl-1H-pyrazolo[3,4-b]pyridin-4-yl)-phenyl]-3-(2-fluorophenyl)-urea
Example 5:
   1-[4-(3-Amino-6-tert-butyl-1 H-pyrazolo[3,4-b]pyridin-4-yl)-phenyl]-3-(2,5-difluorophenyl)-urea
Example 6:
   1-[4-(3-Amino-6-tert-butyl-1 H-pyrazolo[3,4-b]pyridin-4-yl)-phenyl]-3-(3,4-difluorophenyl)-urea
Example 7:
   1-[4-(3-Amino-6-tert-butyl-1 H-pyrazolo[3,4-b]pyridin-4-yl)-phenyl]-3-naphthalen-1-yl-u rea
Example 8:
   1-[4-(3-Amino-6-tert-butyl-1H-pyrazolo[3,4-b]pyridin-4-yl)-phenyl]-3-naphthalen-2-yl-urea
Example 9:
   1-[4-(3-Amino-6-tert-butyl-1H-pyrazolo[3,4-b]pyridin-4-yl)-phenyl]-3-m-tolyl-urea
Example 10:
   1-[4-(3-Amino-6-tert-butyl-1 H-pyrazolo[3,4-b]pyridin-4-yl)-phenyl]-3-(2-nitrophenyl)-urea
Example 11:
   1-[4-(3-Amino-6-tert-butyl-1 H-pyrazolo[3,4-b]pyridin-4-yl)-phenyl]-3-(2,4-difluorophenyl)-urea
Example 12:
   1-[4-(3-Amino-6-tert-butyl-1H-pyrazolo[3,4-b]pyridin-4-yl)-phenyl]-3-(4-fluorophenyl)-urea
Example 13:
   1-[4-(3-Amino-6-tert-butyl-1H-pyrazolo[3,4-b]pyridin-4-yl)-phenyl]-3-(4-cyanophenyl)-urea
Example 14:
   1-[4-(3-Amino-6-cyclopropyl-1 H-pyrazolo[3,4-b]pyridin-4-yl)-phenyl]-3-phenyl-urea
Example 15:
   1-[4-(3-Amino-6-cyclopropyl-1 H-pyrazolo[3,4-b]pyridin-4-yl)-phenyl]-3-(2-fluorophenyl)-urea

Another aspect of the invention is the method of preparation of pyrazolopyrimidines of general formula (I) described hereinbefore, the method comprising the following method steps:
A) an aldehyde of formula 1 is reacted with methylketone of formula 2, an alkyl cyanoacetate and an ammonium acetate into a compound of formula 3 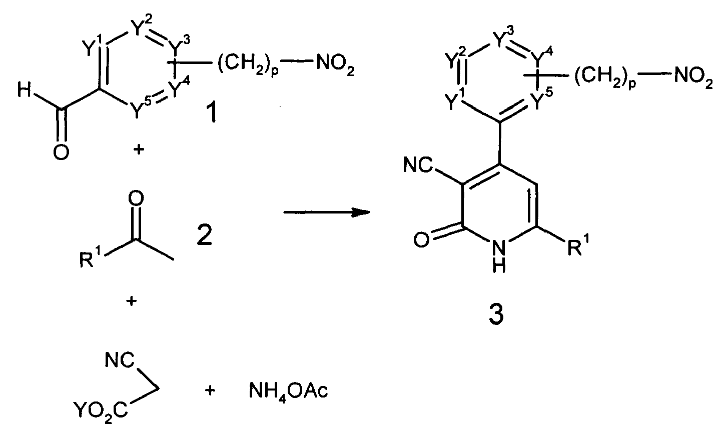
B) a compound of formula 3 reacted to a compound of formula 4 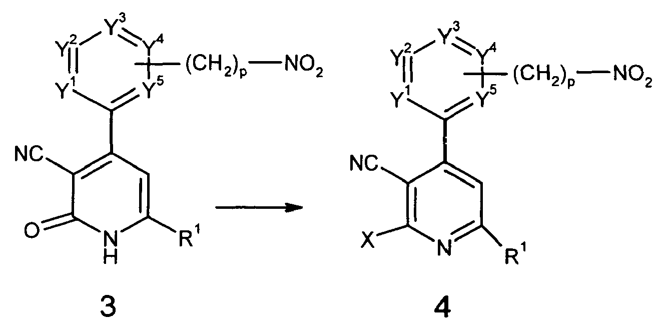
C) a compound of formula 4 reduced to a compound of formula 5 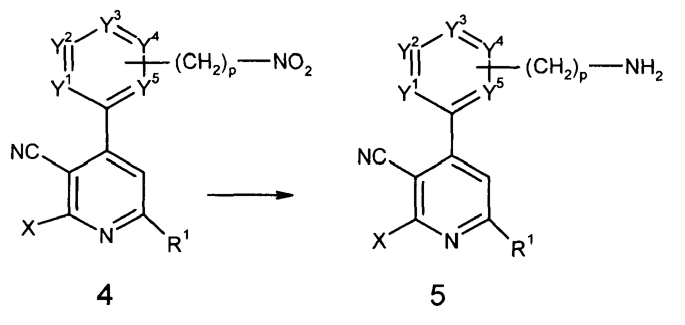
D) a compound of formula 5 reacted to a compound of formula 6 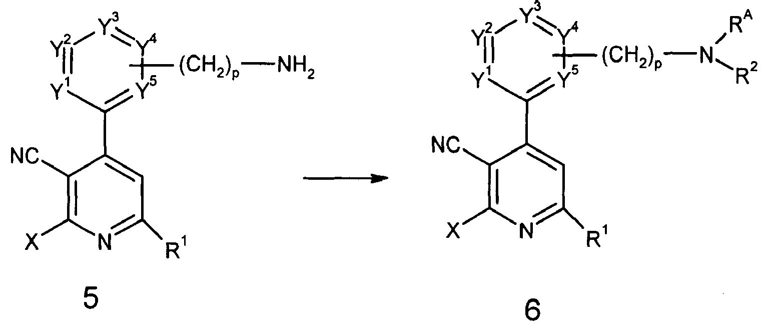
E) a compound of formula 6 reacted by addition of hydrazines to a compound of general formula (I) 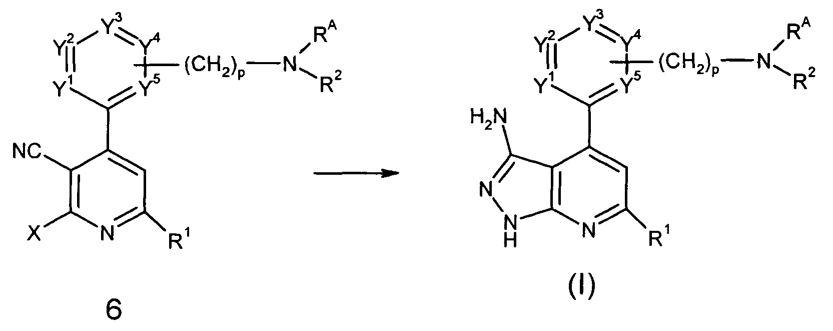
wherein Y stands for C₁-C₄-alkyl, X stands for halogen or perfluor-C₁-C₄-alkyl sulfonyl and R^{A}, R¹, R², Y¹, Y², Y³, Y⁴, Y⁵ and p have the meaning described in general formula (I) hereinbefore as well as N-oxides, solvates, hydrates, isomers, diastereomers, enantiomers and salts thereof.

A further method of preparation of pyrazolopyrimidines of general formula (I) described hereinbefore, is the method comprising the following method steps:
A) a compound of formula 7 reacted to a compound to formula 8 and 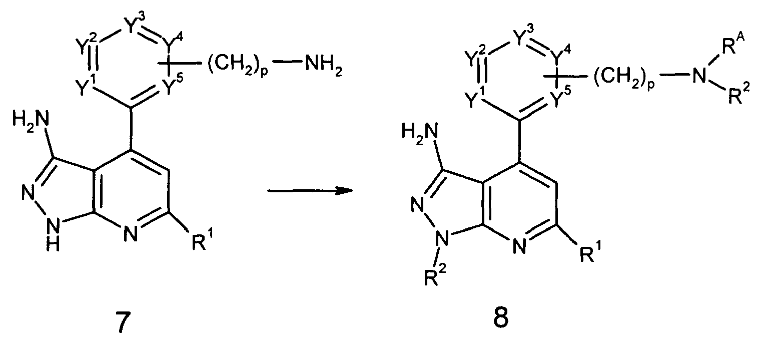
B) a compound of formula 8 reacted to a compound of general formula (I) 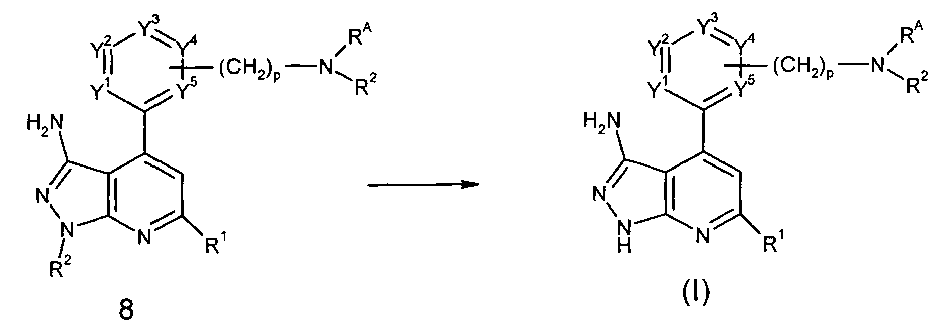
wherein R^{A}, R¹, R², Y¹, Y², Y³, Y⁴, Y⁵ and p have the meaning described in general formula (I) hereinbefore as well as N-oxides, solvates, hydrates, isomers, diastereomers, enantiomers and salts thereof.

The compounds of the present invention can be used in treating diseases of dysregulated vascular growth or diseases which are accompanied with dysregulated vascular growth. Especially the compounds effectively interfere with angiopoietin and therefore influence Tie2 signalling. Surprisingly the compounds block Tie2 signalling, wherein obviously Tie2 kinase activity is blocked. This effect will therefore allow prolonged treatment of patients with the inventive compounds offering good tolerability and high anti-angiogenic efficacy, where persistent angiogenesis plays a pathologic role.

Therefore another aspect of the present invention is a use of the compound of general formula (I) described hereinbefore for manufacturing a pharmaceutical composition for the treatment of diseases of dysregulated vascular growth or of diseases which are accompanied with dysregulated vascular growth.

Preferably the use is in the treatment of diseases, wherein the diseases are tumors and / or metastases thereof.

Another use is in the treatment of diseases, wherein the diseases are retinopathy, other angiogenesis dependent diseases of the eye, in particular cornea transplant rejection or age-related macular degeneration, rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

A further use is in the treatment of diseases, wherein the diseases are coronary and peripheral artery disease.

Another use is in the treatment of diseases, wherein the diseases are ascites, edema like brain tumour associated edema, high altitude trauma, hypoxia induced cerebral edema pulmonary edema and macular edema or edema following burns and trauma, chronic lung disease, adult respiratory distress syndrome, bone resorbtion and for benign proliferating diseases like myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

A further use is in the treatment of diseases, wherein the diseases are retinopathy, other angiogenesis dependent diseases of the eye, rheumatoid arthritis, and other inflammatory diseases associated with angiogenesis.

A next aspect of the invention is a method of treating a disease of dysregulated vascular growth or diseases which are accompanied with dysregulated vascular growth by administering an effective amount of a compound of general formula (I) described hereinbefore.

Preferably the diseases of said method is tumor and / or metastases thereof.

Also the diseases of said method are retinopathy, other angiogenesis dependent diseases of the eye, in particular cornea transplant rejection or age-related macular degeneration e.g. , rheumatoid arthritis, and other inflammatory diseases associated with angiogenesis, in particular psoriasis, delayed type hypersensitivity, contact dermatitis, asthma, multiple sclerosis, restenosis, pulmonary hypertension, stroke, and diseases of the bowel.

Further the disease of the method are coronary and peripheral artery disease.

Another diseases of the method are ascites, edema like brain tumour associated edema, high altitude trauma, hypoxia induced cerebral edema pulmonary edema and macular edema or edema following burns and trauma, chronic lung disease, adult respiratory distress syndrome, bone resorbtion and for benign proliferating diseases like myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

Therefore the compounds of the present invention can be applied for the treatment of diseases accompanied by neoangiogenesis. This holds principally for all solid tumours, e.g. mamma, colon, renal, lung and/or brain tomours or metastases thereof and can be extended to a broad range of diseases, where pathologic angiogenesis is persistent. This applies for diseases with inflammatory association, diseases associated with edema of various forms and diseases associated with stromal proliferation and pathologic stromal reactions broadly. Particularly suited is the treatment for gynaecological diseases where inhibition of angiogenic, inflammatory and stromal processes with pathologic character can be inhibited. At the same time the toxic side effects on normal proliferating tissue are low. The treatment is therefore an addition to the existing armament to treat diseases associated with neoangiogenesis.

The compounds of the present invention can be used in particular in therapy and prevention of tumour growth and metastases especially in solid tumours of all indications and stages with or without pre-treatment if the tumour growth is accompanied with persistent angiogenesis. However it is not restricted to tumour therapy but is also of great value for the treatment of other diseases with dysregulated vascular growth. This includes retinopathy and other angiogenesis dependent diseases of the eye (e.g. cornea transplant rejection, age-related macular degeneration), rheumatoid arthritis, and other inflammatory diseases associated with angiogenesis like psoriasis, delayed type hypersensitivity, contact dermatitis, asthma, multiple sclerosis, restenosis, pulmonary hypertension, stroke and inflammatory diseases of the bowel, like Crohn's disease. It includes coronary and peripheral artery disease. It can be applied for disease states like ascites, edema, like brain tumour associated edema, high altitude trauma, hypoxia induced cerebral edema, pulmonary edema and macular edema or edema following burns and trauma. Furthermore it is useful for chronic lung disease, adult respiratory distress syndrome. Also for bone resorption and for benign proliferating diseases like myoma, benign prostate hyperplasia and wound healing for the reduction of scar formation. It is therapeutically valuable for the treatment of diseases, where deposition of fibrin or extracellular matrix is an issue and stroma proliferation is accelerated (e.g. fibrosis, cirrhosis, carpal tunnel syndrome etc). In addition it can be used for the reduction of scar formation during regeneration of damaged nerves, permitting the reconnection of axons. Further uses are endometriosis, pre-eclampsia, postmenopausal bleeding and ovarian hyperstimulation.

Another aspect of the present invention is a pharmaceutical composition which contains a compound of Formula I or pharmaceutically acceptable salts thereof, N-oxides, solvates, hydrates, isomers or mixtures of isomers thereof, in admixture with one or more suitable excipients. This composition is particularly suited for the treatment of diseases of dysregulated vascular growth or of diseases which are accompanied with dysregulated vascular growth as explained above.

For using the compounds of the present invention as pharmaceutical product the compounds or mixtures thereof are provided in a pharmaceutical composition, which beside the compounds of the present invention for enteral, oral or parenteral application contain suitably pharmaceutically acceptable organic or inorganic inert base material, e.g. purified water, gelatin, rubber arabicum, lactate, starch, magnesium stearate, talcum, vegetable oils, polyalkylenglycole, etc.

The pharmaceutical composition may be provided in a solid form, e.g. as tablets, dragèes, suppositories, capsules or in liquid form, e.g. as a solution, suspension or emulsion. The pharmaceutical composition may additionally contain auxiliary substances, e.g. conservators, stabilisers, wetting agents or emulgators, salts for adjusting the osmotic pressure or buffers.

For parenteral application (including intravenous, subcutaneous, intramuscular, intravascular or infusion) sterile injection solutions or suspensions are preferred, especially aqueous solutions of the compounds in polyhydroxyethoxy containing castor oil.

The pharmaceutical composition may further contain surface active agents, *e.g*. salts of gallenic acid, phosphorlipids of animal or vegetable origin, mixtures thereof and liposomes and parts thereof.

For oral application tablets, dragèes or capsules with talcum and/or carbonhydrogen carriers and -binders, *e.g*. lactose, maize and potato starch, are preferred. Further application in liquid form is possible, for example as juice, which contains sweetener if necessary.

The dosage will necessarily be varied depending upon the route of administration, age, weight of the patient, the kind and severity of the illness being treated and similar factors. The daily dose is in the range of 0.5 bis 1.500 mg. A dose can be administered as unit dose or in part thereof and distributed over the day. Accordingly the optimum dosage may be determined by the practitioner who is treating any particular patient.

Another aspect of the present invention is the method of preparing the compounds according to the present invention.

The following table lists the abbreviations used in this paragraph, and in the Examples section. NMR peak forms are stated as they appear in the spectra, possible higher order effects have not been considered.

| Abbreviation | Meaning |
|---|---|
| Ac | Acetyl |
| Boc | *tert*-butyloxycarbonyl |
| br | broad |
| c- | cyclo- |
| Cl | chemical ionisation |
| d | doublet |
| dd | doublet of doublet |
| DCM | dichloromethane |
| DIPEA | N,N-diisopropylethyl amine |
| DMF | N, N-dimethylformamide |
| DMSO | Dimethyl sulfoxide |
| eq | equivalent |
| ESI | electrospray ionisation |
| GP | general procedure |
| m | multiplet |
| mc | centred multiplet |
| MS | mass spectrometry |
| NMR | nuclear magnetic resonance spectroscopy |
| POPd | Dihydrogen dichlorobis(di-*tert*-butyl phosphinito-κP)palladate(2); CombiPhos Catalysts, Inc. |
| q | quartet |
| s | singlet |
| t | triplet |
| TEA | triethylamine |
| TFA | trifluoroacetic acid |
| THF | tetrahydrofuran |

The following schemes and general procedures illustrate general synthetic routes to the compounds of the invention and are not intended to be limiting. Specific examples are described in the subsequent paragraph.

A first reaction scheme is outlined herein below:

### Synthesis of compounds of general formula (I)

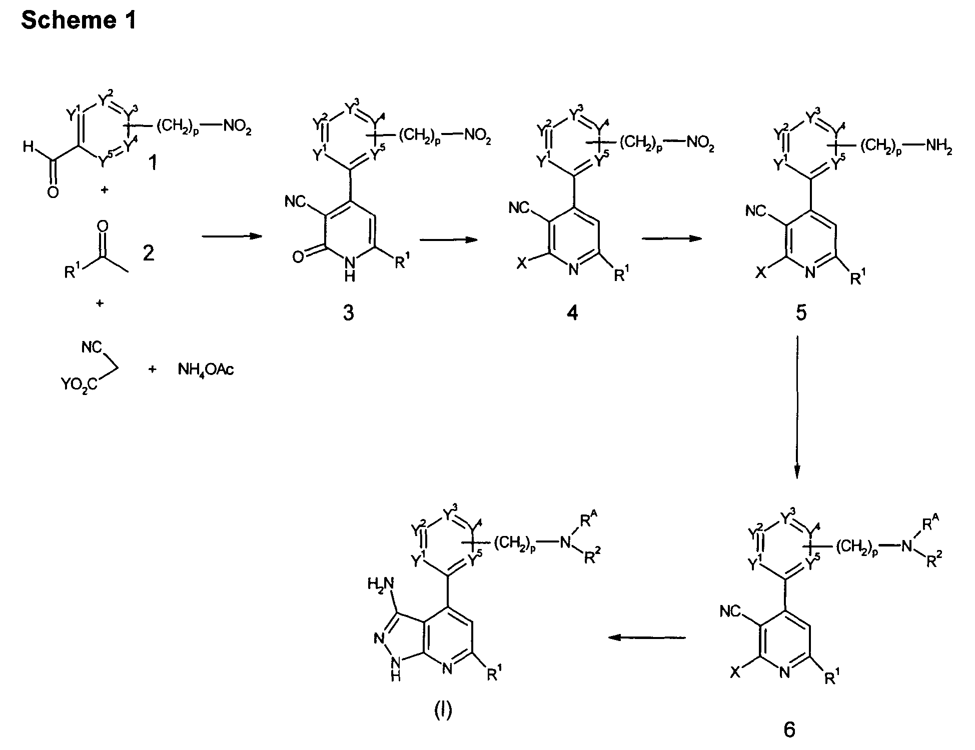

wherein Y stands for C₁-C₄-alkyl, X stands for halogen or perfluor-C₁-C₄-alkyl sulfonyl and R^{A}, R¹, R², Y¹, Y², Y³, Y⁴, Y⁵ and p have the meaning described in general formula (I).

Compounds of general formula (I) can be synthesized according to the procedure depicted in scheme 1. Reaction of aldehyde 1 with methylketone 2, alkyl cyanoacetate, and ammonium acetate yields compounds of formula 3 which are formed into compounds of formula 4. Reduction of the nitro group gives compounds of formula 5. Compounds of formula 6 are formed from compounds of formula 5 by reaction with e.g. aryl- or heteroaryl isocyanates or aryl- or heteroarylsulfonyl chlorides. Addition of hydrazine to compounds of formula 6 leads to compounds of general formula (I). The substituents Y¹, Y², Y³, Y⁴ Y⁵, R¹, R^{A}, R² may be further modified on each step (formula 3 to formula 6) or in the last step (general formula 1). These modifications might be cleavage of protecting groups, reduction or oxydation of functional groups, substitution or other reactions. The sequence shown in scheme 1 can be also changed in a way that modifications or introduction of the substituents is performed earlier or later. In particular the introduction of substituents R^{A} and R² can be performed earlier or later. It is possible, that e.g. aldehydes of type 1 already contain the the group (CH₂)ₚNR^{A}R² instead of (CH₂)ₚNO₂. On the other hand it is also possible e.g. to introduce R² after the pyrazole ring has been formed. In certain cases double addition occurs and compounds of formula 8 (scheme 2) are obtained. In these cases a selective cleavage of the group R² at pyrazole nitrogen 1 can be achieved for example by treatment of 8 with potassium carbonate in methanol to give compounds of general formula (I).

A second reaction scheme is outlined below:

### Synthesis of compounds of general formula (I)

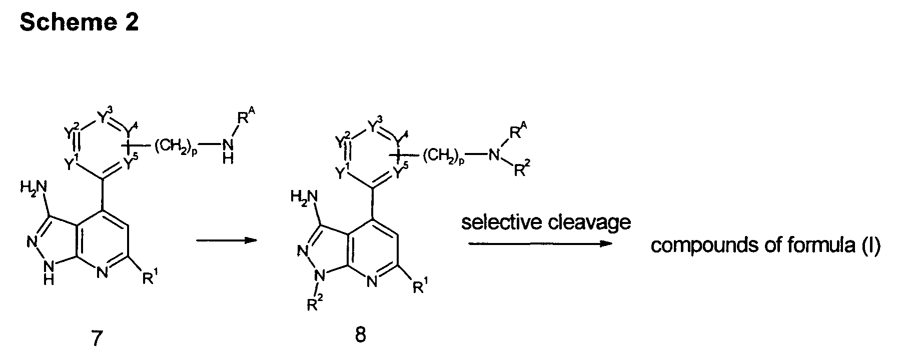

The substituents Y¹, Y², Y³, Y⁴, Y⁵, R¹, R^{A}, R² and p have the same meaning as described in the general formula 1. In addition functional groups may be protected. Possible protecting groups are ketals/acetals, esters, amides, nitro groups, carbamates, alkyl ethers, allyl ethers, benzyl ethers, or silyl ethers such as e.g. trimethylsilyl, tert-butyldimethylsilyl, tert-butyl-diphenylsilyl, triethylsilyl.

### Example 1 Preparation of 1-[4-(3-Amino-6-tert-butyl-1H-pyrazolo[3,4-b]pyridin-4-yl)-phenyl]-3-phenyl-urea

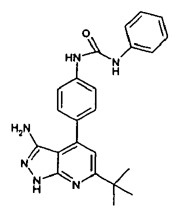

### Example 1a Preparation of 1-(4-Hydroxymethyl-phenyl)-3-phenylurea

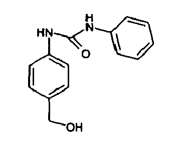

A solution of 3.75 g 4-Aminobenzylalkohol and 3.3 ml lsocyanato-benzene in 120 ml dichloromethane was stirred at 23°C for 2 hours. Afterwards, the reaction mixture was filtered. The crystalline product was washed with diethylether. 6.82 g product was obtained which was used without further purification.
¹H-NMR (d6-DMSO): δ = 4,40 (2H); 5,06 (1H); 6,96 (1H); 7,16-7,32 (4H); 7,35-7,52 (4H); 8,63 (2H) ppm.

### Example 1b Preparation of 1-(4-Formyl-phenyl)-3-phenyl-urea

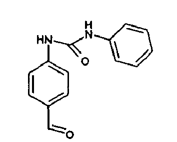

A solution of 3.41 g 1 a and 10 g manganese dioxide in toluene was stirred at 23°C for 20 hours. Afterwards, the reaction mixture was filtered through celite and evaporated in vacuo. The crude product (2.57 g) was used without further purification.
¹H-NMR (d6-DMSO): δ = 7,00 (1H); 7,30 (2H); 7,48 (2H); 7,67 (2H); 7,84 (2H); 8,87 (1 H); 9,21 (1 H) 9,85 (1 H) ppm.

### Example 1 c Preparation of 1-[4-(6-tert-Butyl-3-cyano-2-oxo-1,2-dihydro-pyridin-4-yl)-phenyl]-3-phenyl-urea

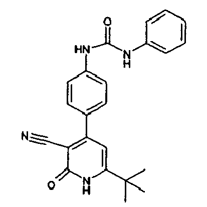

A solution of 6.66 g ammonium acetate, 1.14 ml methyl cyanoacetate, 1.32 ml 3,3-dimethylbutan-2-one, and 2.57 g 1b in 100 ml ethanol was stirred at 80°C for 6 hours. Afterwards, it was cooled to 23°C and the precipitated product was obtained by filtration. The product was purified by recrystallization from methylene chloride/ methanol. 1.44 g product was isolated.
¹H-NMR (d6-DMSO): δ = 1,30 (9H); 6,25 (2H); 6,99 (1 H); 7,29 (2H); 7,46 (2H); 7,13 (4H); 8,79 (1 H); 9,02 (1 H); 12,18 (1 H) ppm.

### Example 1d Preparation of Trifluoromethanesulfonic acid 6-tert-butyl-3-cyano-4-[4-(3-phenyl-ureido)-phenyl]-pyridin-2-yl ester

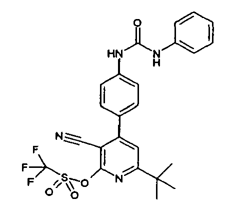

A solution of 340 mg 1 c and 300 µl trifluoromethanesulfonic acid anhydride in pyridine was stirred at 0°C for 3 hours. Afterwards, the reaction mixture was poured into a saturated aqueous sodium hydrogen carbonate solution. It was extracted with ethyl acetate and the organic layer was washed with brine. It was dried over sodium sulfate and evaporated in vacuo. The crude product was purified by column chromatography on silica gel. 116 mg product was obtained.
¹H-NMR (d6-DMSO): δ = 1,36 (9H); 7,00 (1H); 7,29 (2H); 7,47 (2H); 7,65-7,85 (5H); 8,80 (1 H); 9,07 (1 H) ppm.

### Example 1 e Preparation of 1-[4-(3-Amino-6-tert-butyl-1 H-pyrazolo[3,4-b]pyridin-4-yl)-phenyl]-3-phenyl-urea

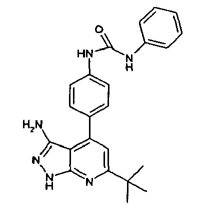

A solution of 78 mg 1d and 30 µl hydrazine hydrate (80%) in propanol was stirred at 100°C for 3 hours. Afterwards, the reaction mixture was cooled to 0°C and left at this temperature for 2 hours. Then, the precipitated product was isolated by filtration. The product was washed with ice-cold propanol and dried at 50°C in vacuo. The crude product was purified by column chromatography on silica gel. 25 mg product was isolated.
¹H-NMR (d6-DMSO): δ = 1,39 (9H); 4,52 (2H); 6,93-7,03 (2H); 7,29 (2H); 7,44-7,58 (4H); 7,65 (2H); 8,81 (1H); 8,99 (1H); 12,12 (1H) ppm.

### Example 2 Preparation of N-[4-(3-Amino-6-tert-butyl-1H-pyrazolo[3,4-b]pyridin-4-yl)-phenyl]-benzenesulfonamide

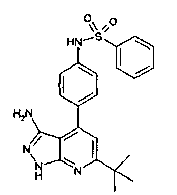

### Example 2a Preparation of N-(4-Hydroxymethyl-phenyl)-benzenesulfonamide

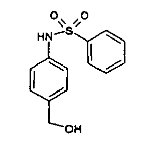

A solution of 500 mg (4-aminophenyl)-methanol, 1.7 ml triethylamine, and 780 µl benzenesulfonyl chloride in 10 ml tetrahydrofuran was stirred at 23°C for 17 hours. Afterwards, the reaction mixture was poured into ice-old hydrochloric acid. It was stirred for 10 minutes and then extracted with ethyl acetate. The organic layer was washed with brine and dried over sodium sulfate. The crude product was purified by column chromatography on silica gel. 572 mg product was isolated.
¹H-NMR (CDCl₃): δ = 4,62 (2H); 6,68 (2H); 7,05 (2H); 7,24 (2H); 7,40-7,48 (2H); 7,54 (1 H); 7,77 (2H) ppm.

### Example 2b Preparation of N-(4-Formyl-phenyl)-benzenesulfonamide

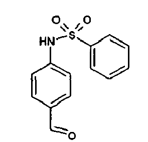

A solution of 303 mg 2a, 260 mg 4-methyl-morpholine 4-oxide, and 22 mg tetrapropylammonium peruthenate in dichloromethane was stirred with molecular sieves (4 A) at 23°C for 17 hours. Afterwards, it was filtered and the filtrate was evaporated in vacuo. The crude product was purified by column chromatography on silica gel. 234 mg product was isolated.
¹H-NMR (CDCl₃): δ = 7,23 (2H); 7,35-7,65 (4H); 7,78 (2H); 7,89 (2H); 9,89 (1H) ppm.

### Example 2c Preparation of N-[4-(6-tert-Butyl-3-cyano-2-oxo-1, 2-dihydro-pyridin-4-yl)-phenyl]-benzenesulfonamide

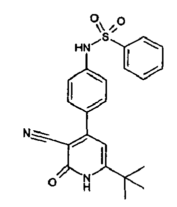

In analogy to the procedure described for example 1c reaction of 1.16 g ammonium acetate, 200 µl methyl cyanoacetate, 235 µl 3,3-dimethylbutan-2-one, and 492 mg of 2b yielded 237 mg product.
¹H-NMR (d6-DMSO): δ = 1,26 (9H); 6,18 (1H); 7,23 (2H); 7,52-7,70 (5H); 7,86 (2H); 10,80 (1H); 12,22 (1H) ppm.

### Example 2d Preparation of Trifluoromethanesulfonic acid 4-(4-benzenesulfonylamino-phenyl)-6-tert-butyl-3-cyano-pyridin-2-yl ester

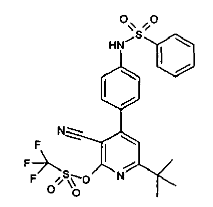

In analogy to the procedure described for example 1 d reaction of 193 mg 2c and 240 µl trifluoromethanesulfonic acid anhydride in pyridine yielded 219 mg product.
¹H-NMR (CDCl₃): δ = 1,36 (9H); 7,22-7,35 (4H); 7,45-7,60 (2H); 7,69 (1H); 7,86 (1 H); 8,62 (2H) ppm.

### Example 2e Preparation of N-[4-(3-Amino-6-tert-butyl-1 H-pyrazolo[3,4-b]pyridin-4-yl)-phenyl]-benzenesulfonamide

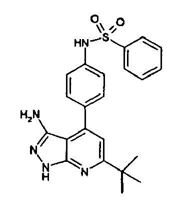

In analogy to the procedure described for example 1e reaction of 212 mg 2d and 72 µl hydrazine hydrate in propanol yielded 69 mg product.
¹H-NMR (d6-DMSO): δ = 1,33 (9H); 4,40 (2H); 6,89 (1 H); 7,25 (2H); 7,46 (2H); 7,52-7,68 (3H); 7,84 (2H); 10,52 (1H); 12,12 (1H) ppm.

### Example 3 Preparation of 1-[4-(3-Amino-6-tert-butyl-1H-pyrazolo[3,4-b]pyridin-4-yl)-phenyl]-3-(3-fluoro-phenyl)-urea

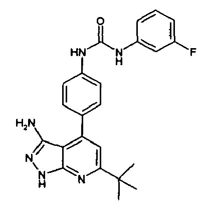

### Example 3a Preparation of 6-tert-Butyl-4-(4-nitro-phenyl)-2-oxo-1,2-dihydropyridine-3-carbonitrile

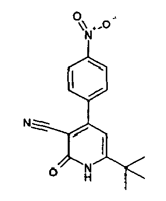

In analogy to the procedure described for example 1 c reaction of 41 g ammonium acetate, 7 ml methyl cyanoacetate, 8.2 ml 3,3-dimethylbutan-2-one, and 10 g 4-nitrobenzaldehyde yielded 4.71 g product.
¹H-NMR (d6-DMSO): δ = 1,32 (9H); 6,30 (1H); 7,92 (2H); 8,39 (2H) ppm.

### Example 3b Preparation of Trifluoromethanesulfonic acid 6-tert-butyl-3-cyano-4-(4-nitro-phenyl)-pyridin-2-yl ester

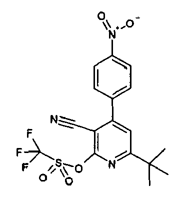

In analogy to the procedure described for example 1 d reaction of 2.5 g 3a and 4.25 ml Trifluoromethanesulfonic acid anhydride in pyridine yielded 3.01 g product.
¹H-NMR (CDCl₃): δ = 1,41 (9H); 7,48 (1H); 7,78 (2H); 8,42 (2H) ppm.

### Example 3c Preparation of Trifluoro-methanesulfonic acid 4-(4-amino-phenyl)-6-tert-butyl-3-cyano-pyridin-2-yl ester

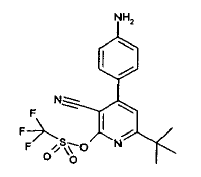

A solution of 3 g 3b and 8 g tin(II) chloride dihydrate in 50 ml ethanol was stirred at 70°C for 10 minutes. Afterwards, the reaction mixture was poured into 120 ml of an aqueous solution of ammonia (33%). It was stirred for 45 minutes at 23°C. Afterwards, it was extracted with ethyl acetate. The organic layer was washed with brine, dried over sodium sulfate and evaporated in vacuo. The crude product (2.6 g) was used without further purification.
¹H-NMR (d6-DMSO): δ = 1,32 (9H); 5,96 (2H); 6,71 (2H); 7,54 (2H); 7,65 (1 H) ppm.

### Example 3d Preparation of Trifluoromethanesulfonic acid 6-tert-butyl-3-cyano-4-{4-[3-(3-fluoro-phenyl)-ureido]-phenyl}-pyridin-2-yl ester

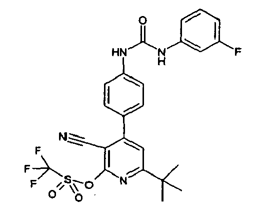

In analogy to the procedure described for example 1 a reaction of 200 mg 3c and 60 µl 1-Fluoro-3-isocyanato-benzene yielded 260 mg product.
¹H-NMR (d6-DMSO): δ = 1,35 (9H); 6,80 (1 H); 7,14 (1 H); 7,30 (1 H); 7,49 (1 H); 7,66-7,85 (5H); 8,96 (1 H); 9,13 (1 H) ppm.

### Example 3e Preparation of 1-[4-(3-Amino-6-tert-butyl-1 H-pyrazolo[3,4-b]pyridin-4-yl)-phenyl]-3-(3-fluoro-phenyl)-urea

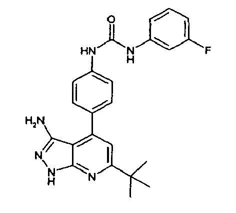

In analogy to the procedure described for example 1e reaction of 260 mg 3d with 91 µl hydrazine hydrate in propanol yielded 141 mg product.
¹H-NMR (d6-DMSO): δ = 1,38 (9H); 4,50 (2H); 6,79 (1 H); 6,96 (1 H); 7,15 (1 H); 7,31 (1 H); 7,49-7,60 (3H); 7,65 (2H); 8,98 (2H); 12,11 (1 H) ppm.

### Example 4 Preparation of 1-[4-(3-Amino-6-tert-butyl-1H-pyrazolo[3,4-b]pyridin-4-y))-phenyl]-3-(2-fluoro-phenyl)-urea

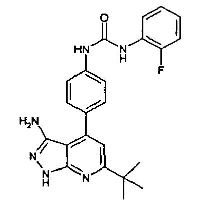

### Example 4a Preparation of Trifluoromethanesulfonic acid 6-tert-butyl-3-cyano-4-{4-[3-(2-fluoro-phenyl)-ureido]-phenyl}-pyridin-2-yl ester

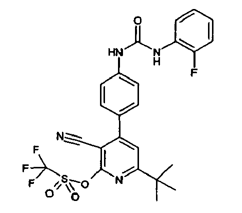

In analogy to the procedure described for example 1 a reaction of 200 mg 3c and 60 µl 1-Fluoro-2-isocyanato-benzene yielded 201 mg product.
¹H-NMR (d6-DMSO): δ = 1,35 (9H); 7,03 (1 H); 7,10-7,30 (2H); 7,69 (2H); 7,78 (3H); 8,16 (1 H); 8,70 (1 H); 9,45 (1 H) ppm.

### Example 4b Preparation of 1-[4-(3-Amino-6-tert-butyl-1 H-pyrazolo[3,4-b]pyridin-4-yl)-phenyl]-3-(2-fluoro-phenyl)-urea

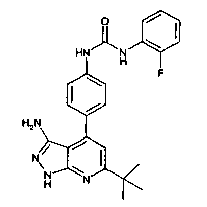

In analogy to the procedure described for example 1e reaction of 196 mg 4a with 70 µl hydrazine hydrate in propanol yielded 61 mg product.
¹H-NMR (d6-DMSO): δ = 1,38 (9H); 4,50 (2H); 6,95 (1 H); 7,04 (1 H); 7,16 (1 H); 7,26 (1 H); 7,55 (2H); 7,66 (2H); 8,18 (1 H); 8,62 (1 H); 9,31 (1 H); 12,10 (1 H) ppm.

### Example 5 Preparation of 1-[4-(3-Amino-6-tert-butyl-1H-pyrazolo[3,4-b]pyridin-4-yl)-phenyl]-3-(2,5-difluoro-phenyl)-urea

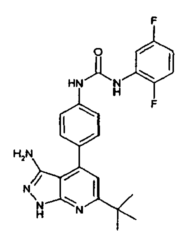

### Example 5a Preparation of Trifluoromethanesulfonic acid 6-tert-butyl-3-cyano-4-{4-[3-(2,5-difluoro-phenyl)-ureido]-phenyl}-pyridin-2-yl ester

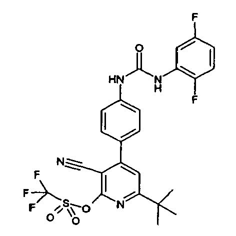

In analogy to the procedure described for example 1a reaction of 200 mg 3c and 71 µl 1- 1,4-Difluoro-2-isocyanato-benzene yielded 264 mg product.
¹H-NMR (d6-DMSO): δ = 1,36 (9H); 6,85 (1H); 7,31 (1H); 7,70 (2H); 7,79 (3H); 8,04 (1 H); 8,90 (1 H); 9,50 (1 H) ppm.

### Example 5b Preparation of 1-[4-(3-Amino-6-tert-butyl-1H-pyrazolo[3,4-b]pyridin-4-yl)-phenyl]-3-(2,5-difluoro-phenyl)-urea

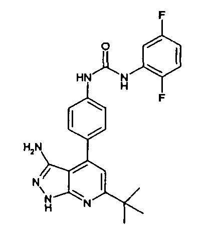

In analogy to the procedure described for example 1e reaction of 261 mg 5a with 90 µl hydrazine hydrate in propanol yielded 63 mg product.
¹H-NMR (d6-DMSO): *δ = 1,39 (9H); 4,51 (2H); 6,84 (1 H); 6,96 (1 H); 7,31 (1 H); 7,58 (2H); 7,66 (2H); 8,06 (1 H); 8,84 (1 H); 9,38 (1 H); 21,10 (1 H) ppm.

### Example 6 Preparation of 1-[4-(3-Amino-6-tert-butyl-1H-pyrazolo[3,4-b]pyridin-4-yl)-phenyl]-3-(3,4-difluoro-phenyl)-urea

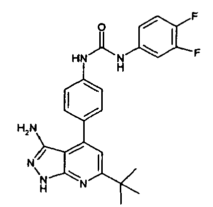

### Example 6a Preparation of Trifluoromethanesulfonic acid 6-tert-butyl-3-cyano-4-{4-[3-(3,4-difluoro-phenyl)-ureido]-phenyl}-pyridin-2-yl ester

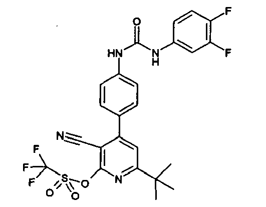

In analogy to the procedure described for example 1a reaction of 200 mg 3c and 71 µl 1,2-Difluoro-4-isocyanato-benzene yielded 275 mg product.
¹H-NMR (d6-DMSO): δ = 1,31 (9H); 7,12 (1H); 7,32 (1H); 7,60-7,78 (6H); 9,02 (1 H); 9,14 (1 H) ppm.

### Example 6b Preparation of 1-[4-(3-Amino-6-tert-butyl-1H-pyrazolo[3,4-b]pyridin-4-yl)-phenyl]-3-(3,4-difluoro-phenyl)-urea

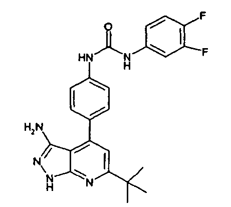

In analogy to the procedure described for example 1e reaction of 270 mg 6a with 91 µl hydrazine hydrate in propanol yielded 153 mg product.
¹H-NMR (d6-DMSO): δ = 1,32 (9H); 4,46 (2H); 6,91 (1H); 7,11 (1H); 7,31 (1H); 7,50 (2H); 7,58-7,70 (3H) 8,95 (2H) ppm.

### Example 7 Preparation of 1-[4-(3-Amino-6-tert-butyl-1 H-pyrazolo(3,4-b]pyridin-4-yl)-phenyl]-3-naphthalen-I -yl-urea

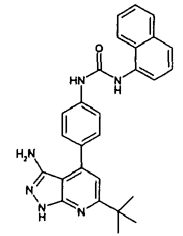

### Example 7a Preparation of Trifluoromethanesulfonic acid 6-tert-butyl-3-cyano-4-[4-(3-naphthalen-1-yl-ureido)-phenyl]-pyridin-2-yl ester

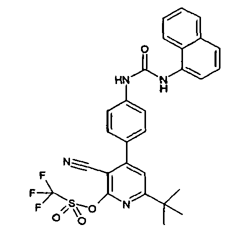

In analogy to the procedure described for example 1a reaction of 200 mg 3c and 80 µl 1-isocyanato-naphthalene yielded 283 mg product.
¹H-NMR (d6-DMSO): δ = 1,31 (9H); 7,45 (1 H); 7,50-7,62 (2H); 7,65 (1 H); 7,70-7,80 (5H); 7,91 (1 H); 7,98 (1 H); 8,10 (1 H); 8,88 (1 H); 9,41 (1 H) ppm.

### Example 7b Preparation of 1-[4-(3-Amino-6-tert-butyl-1 H-pyrazolo[3,4-b]pyridin-4-yl)-phenyl]-3-naphthalen-1-yl-urea

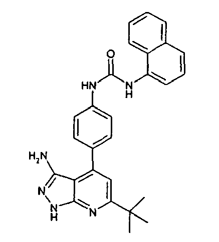

In analogy to the procedure described for example 1e reaction of 280 mg 7a with 91 µl hydrazine hydrate in propanol yielded 137 mg product.
¹H-NMR (d6-DMSO): δ = 1,39 (9H); 4,51 (2H); 6,98 (1H); 7,45-7,78 (8H); 7,95 (1 H); 8,02 (1 H); 8,16 (1 H); 8,84 (1 H); 9,30 (1 H); 12,12 (1 H) ppm.

### Example 8 Preparation of 1-[4-(3-Amino-6-tert-butyl-1H-pyrazolo[3,4-b]pyridin-4-yl)-phenyl]-3-naphthalen-2-yl-urea

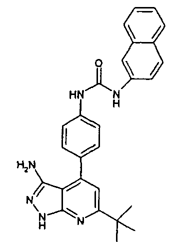

### Example 8a Preparation of Trifluoromethanesulfonic acid 6-tert-butyl-3-cyano-4-[4-(3-naphthalen-2-yl-ureido)-phenyl]-pyridin-2-yl ester

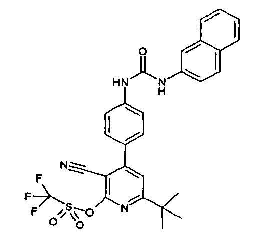

In analogy to the procedure described for example 1 a reaction of 200 mg 3c and 93 mg 2-isocyanato-naphthalene yielded 234 mg product.
¹H-NMR (d6-DMSO): 8 = 1,32 (9H); 7,36 (1 H); 7,45 (1 H); 7,51 (1 H); 7,70-7,90 (8H); 8,12 (1 H); 9,06 (1 H); 9,19 (1 H) ppm.

### Example 8b Preparation of 1-[4-(3-Amino-6-tert-butyl-1 H-pyrazolo[3,4-b]pyridin-4-yl)-phenyl]-3-naphthalen-2-yl-urea

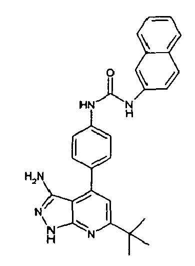

In analogy to the procedure described for example 1e reaction of 227 mg 8a with 75 µl hydrazine hydrate in propanol yielded 131 mg product. ¹H-NMR (d6-DMSO): δ = 1,40 (9H); 4,52 (2H); 6,98 (1 H); 7,38 (1 H); 7,48 (1 H); 7,56 (3H); 7,70 (2H); 7,78-7,90 (3H); 8,13 (1 H); 9,00 (1 H); 9,05 (1 H); 12,12 (1 H) ppm.

### Example 9 Preparation of 1-[4-(3-Amino-6-tert-butyl-1H-pyrazolo[3,4-b]pyridin-4-yl)-phenyl]-3-m-tolyl-urea

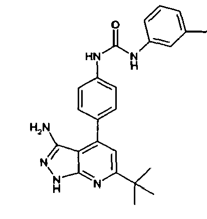

### Example 9a Preparation of Trifluoromethanesulfonic acid 6-tert-butyl-3-cyano-4-[4-(3-m-tolyl-ureido)-phenyl]-pyridin-2-yl ester

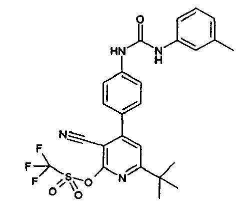

In analogy to the procedure described for example 1a reaction of 200 mg 3c and 70 µl 1-isocyanato-3-methyl-benzene yielded 244 mg product.
¹H-NMR (d6-DMSO): δ = 1,38 (9H); 2,30 (3H); 6,71 (1H); 7,12-7,28 (2H); 7,32 (1 H); 7,64-7,83 (5H); 8,71 (1 H); 9,03 (1 H) ppm.

### Example 9b Preparation of 1-[4-(3-Amino-6-tert-butyl-1 H-pyrazolo[3,4-b]pyridin-4-yl)-phenyl]-3-m-tolyl-urea

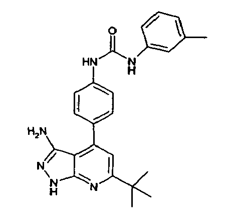

In analogy to the procedure described for example 1e reaction of 237 mg 9a with 81 µl hydrazine hydrate in propanol yielded 113 mg product.
¹H-NMR (d6-DMSO): δ = 1,32 (9H); 2,23 (3H); 4,48 (2H); 6,77 (1H); 6,91 (1H); 7,12 (1 H); 7,22 (1 H); 7,28 (1 H); 7,50 (2H); 7,60 (2H); 8,64 (1 H); 8,89 (1 H); 12,08 (1 H) ppm.

### Example 10 Preparation of 1-[4-(3-Amino-6-tert-butyl-1H-pyrazolo[3,4-b]pyridin-4-yl)-phenyl]-3-(2-nitro-phenyl)-urea

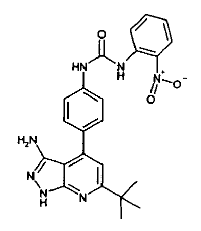

### Example 10a Preparation of Trifluoromethanesulfonic acid 6-tert-butyl-3-cyano-4-{4-[3-(2-nitro-phenyl)-ureido]-phenyl}-pyridin-2-yl ester

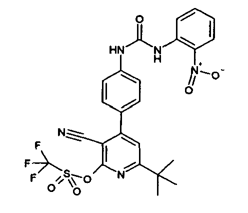

In analogy to the procedure described for example 1a reaction of 200 mg 3c and 91 mg 1-Isocyanato-2-nitro-benzene yielded 275 mg product.
¹H-NMR (d6-DMSO): δ = 1,35 (9H); 7,23 (1H); 7,70-7,86 (6H); 8,11 (1H); 8,30 (1H); 9,67 (1H); 10,17 (1 H) ppm.

### Example 10b Preparation of 1-[4-(3-Amino-6-tert-butyl-1 H-pyrazolo[3,4-b]pyridin-4-yl)-phenyl)-3-(2-nitro-phenyl)-urea

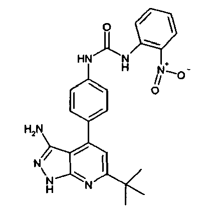

In analogy to the procedure described for example 1e reaction of 270 mg 10a with 88 µl hydrazine hydrate in propanol yielded 97 mg product.
¹H-NMR (d6-DMSO): δ = 1,38 (9H); 4,50 (2H); 6,97 (1 H); 7,22 (1 H); 7,56 (2H); 7,66-7,78 (3H); 8,11 (1 H); 8,31 (1 H); 9,65 (1 H); 10,06 (1 H); 12,10 (1 H) ppm.

### Example 11 Preparation of 1-[4-(3-Amino-6-tert-butyl-1H-pyrazolo[3,4-b]pyridin-4-yl)-phenyl]-3-(2,4-difluoro-phenyl)-urea

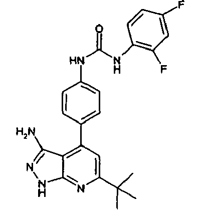

### Example 11 a Preparation of Trifluoro-methanesulfonic acid 6-tert-butyl-3-cyano-4-{4-[3-(2,4-difluoro-phenyl)-ureido]-phenyl}-pyridin-2-yl ester

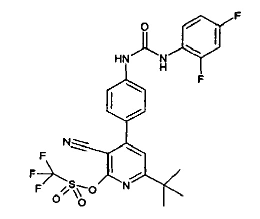

In analogy to the procedure described for example 1a reaction of 200 mg 3c and 71 µl 2,4-Difluoro-1-isocyanato-benzene yielded 259 mg product.
¹H-NMR (d6-DMSO): δ = 1,35 (9H); 7,08 (1H); 7,32 (1H); 7,69 (2H); 7,78 (3H); 8,09 (1 H); 8,65 (1 H); 9,40 (1 H) ppm.

### Example 11 b Preparation of 1-[4-(3-Amino-6-tert-butyl-1 H-pyrazolo[3,4-b]pyridin-4-yl)-phenyl]-3-(2,4-difluoro-phenyl)-urea

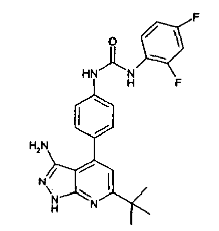

In analogy to the procedure described for example 1e reaction of 254 mg 11a with 85 µl hydrazine hydrate in propanol yielded 58 mg product.
¹H-NMR (d6-DMSO): δ= 1,37 (9H); 4,50 (2H); 6,96 (1H); 7,07 (1H); 7,32 (1H); 7,55 (2H); 7,63 (2H); 8,10 (1 H); 8,57 (1 H); 9,22 (1 H); 12,10 (1 H) ppm.

### Example 12 Preparation of 1-[4-(3-Amino-6-tert-butyl-1H-pyrazolo[3,4-b]pyridin-4-yl)-phenyl]-3-(4-fluoro-phenyl)-urea

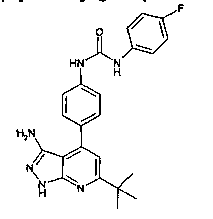

### Example 12a Preparation of Trifluoromethanesulfonic acid 6-tert-butyl-3-cyano-4-{4-[3-(4-fluorophenyl)-ureido]-phenyl}-pyridin-2-yl ester

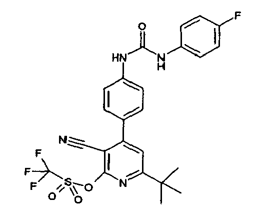

In analogy to the procedure described for example 1a reaction of 200 mg 3c and 70 µ 4-fluoro-1-isocyanato-benzene yielded 264 mg product.
¹H-NMR (d6-DMSO): δ = 1,34 (9H); 7,14 (2H); 7,48 (2H); 7,65-7,82 (5H); 8,83 (1 H); 9,06 (1 H) ppm.

### Example 12b Preparation of 1-[4-(3-Amino-6-tert-butyl-1H-pyrazolo[3,4-b]pyridin-4-yl)-phenyl]-3-(4-fluoro-phenyl)-urea

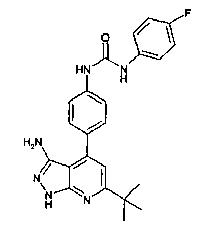

In analogy to the procedure described for example 1e reaction of 259 mg 12a with 88 µl hydrazine hydrate in propanol yielded 127 mg product.
¹H-NMR (d6-DMSO): δ = 1,36 (9H); 4,46 (2H); 6,91 (1H); 7,10 (2H); 7,40-7,53 (4H); 7,60 (2H); 8,74 (1 H); 8,86 (1 H); 12,08 (1 H) ppm.

### Example 13 Preparation of 1-[4-(3-Amino-6-tert-butyl-1H-pyrazolo[3,4-b]pyridin-4-yl)-phenyl]-3-(4-cyano-phenyl)-urea

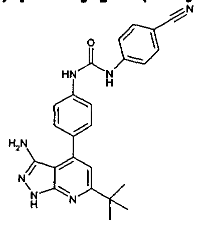

### Example 13a Preparation of Trifluoro-methanesulfonic acid 6-tert-butyl-3-cyano-4-{4-[3-(4-cyano-phenyl)-ureido]-phenyl}-pyridin-2-yl ester

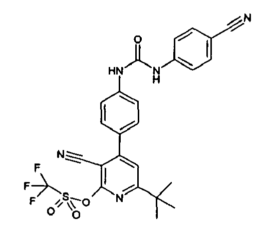

In analogy to the procedure described for example 1a reaction of 200 mg 3c and 90 µl 4-isocyanato-benzonitrile yielded 142 mg product.
¹H-NMR (d6-DMSO): δ = 1,35 (9H); 7,62-7,83 (9H); 9,23 (1 H); 9,32 (1 H) ppm.

### Example 13b Preparation of 1-[4-(3-Amino-6-tert-butyl-1 H-pyrazolo[3,4-b]pyridin-4-yl)-phenyl]-3-(4-cyano-phenyl)-urea

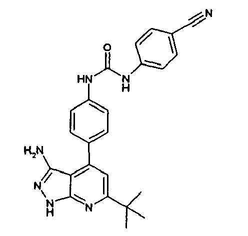

In analogy to the procedure described for example 1e reaction of 259 mg 13a with 50 µl hydrazine hydrate in propanol yielded 53 mg product.
¹H-NMR (d6-DMSO): δ = 1,32 (9H); 4,48 (2H); 6,91 (1H); 7,51 (2H); 7,61 (4H); 7,70 (2H); 9,07 (1 H); 9,24 (1 H); 12,10 (1 H) ppm.

### Example 14 Preparation of 1-[4-(3-Amino-6-cyclopropyl-1H-pyrazolo[3,4-b]pyridin-4-yl)-phenyl]-3-phenyl-urea

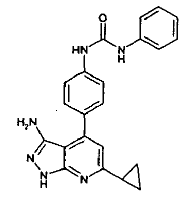

### Example 14a Preparation of 6-Cyclopropyl-4-(4-nitro-phenyl)-2-oxo-1,2-dihydropyridine-3-carbonitrile

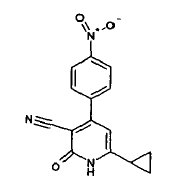

In analogy to the procedure described for example 3a reaction of 17.9 g ammonium acetate, 3.5 ml methyl cyanoacetate, 3.8 ml 1-Cyclopropylethanone, and 5 g 4-nitrobenzaldehyde yielded 3.23 g product.
¹H-NMR (d6-DMSO): δ = 1,00-1,25 (4H); 2,00 (1H); 6,10 (1H); 7,88 (2H); 8,37 (2H); 12,82 (1H) ppm.

### Example 14b Preparation of Trifluoro-methanesulfonic acid 3-cyano-6-cyclopropyl-4-(4-nitro-phenyl)-pyridin-2-yl ester

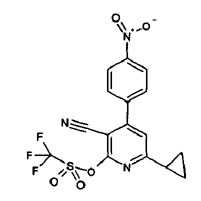

In analogy to the procedure described for example 3b reaction of 1.5 g 14a and 2.69 ml Trifluoromethanesulfonic acid anhydride in pyridine yielded 1.49 g product.
¹H-NMR (CDCl₃): δ = 1,26 (4H); 2,15 (1H); 7,40 (1H); 7,78 (2H); 8,41 (2H) ppm.

### Example 14c Preparation of Trifluoro-methanesulfonic acid 4-(4 -amino-phenyl)-3-cyano-6-cyclopropyl-pyridin-2-yl ester

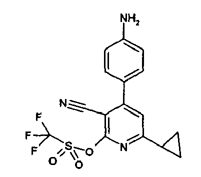

In analogy to the procedure described for example 3c reaction of 1.49 g 14b and 4.1 g tin(II) chloride dihydrate in ethanol yielded 1.2 g product.
¹H-NMR (d6-DMSO): δ = 0,98 (2H); 1,17 (2H); 2,31 (1H); 5,92 (2H); 6,70 (2H); 7,51 (2H); 7,76 (1 H) ppm.

### Example 14d Preparation of Trifluoromethanesulfonic acid 3-cyano-6-cyclopropyl-4-[4-(3-phenyl-ureido)-phenyl]-pyridin-2-yl ester

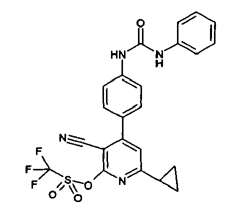

In analogy to the procedure described for example 3d reaction of 250 mg 14c and 70 µl Isocyanato-benzene yielded 266 mg product.
¹H-NMR (d6-DMSO): δ = 1,03 (2H); 1,21 (2H); 2,37 (1H); 6,99 (1H); 7,30 (2H); 7,48 (2H); 7,65-7,77 (4H); 7,86 (1H); 8,86 (1H); 9,10 (1H); 1,36 (9H); 7,22-7,35 (4H); 7,45-7,60 (2H); 7,69 (1H); 7,86 (1H); 8,62 (2H) ppm.

### Example 14e Preparation of 1-[4-(3-Amino-6-cyclopropyl-1 H-pyrazolo[3,4-b]pyridin-4-yl)-phenyl]-3-phenyl-urea

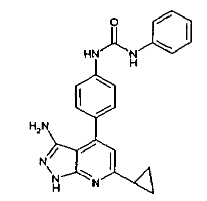

In analogy to the procedure described for example 3e reaction of 261 mg 14d with 95 µl hydrazine hydrate in propanol yielded 78 mg product.
¹H-NMR (d6-DMSO): δ = 1,00 (4H); 2,19 (1H); 4,48 (2H); 6,86 (1H); 6,99 (1H); 7,30 (2H); 7,45-7,59 (4H); 7,63 (2H); 8,78 (1 H); 8,91 (1 H); 11,97 (1 H) ppm.

### Example 15 Preparation of 1-[4-(3-Amino-6-cyclopropyl-1H-pyrazolo[3,4-b]pyridin-4-yl)-phenyl]-3-(2-fluoro-phenyl)-urea

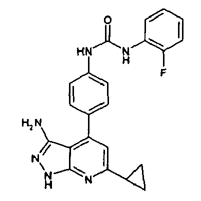

### Example 15a Preparation of Trifluoromethanesulfonic acid 3-cyano-6-cyclopropyl-4-{4-[3-(2-fluorophenyl)-ureido]-phenyl}-pyridin-2-yl ester

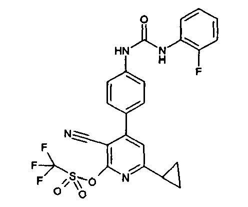

In analogy to the procedure described for example 3d reaction of 200 mg 14c and 71 µl 1-Fluoro-2-isocyanato-benzene yielded 179 mg product.
¹H-NMR (d6-DMSO): δ = 0,99 (2H); 1,19 (2H); 2,32 (1H); 7,00 (1H); 7,11 (1H); 7,21 (1H); 7,61-7,74 (4H); 7,83 (1H); 8,10 (1H); 8,69 (1H); 9,42 (1H) ppm.

### Example 15b Preparation of 1-[4-(3-Amino-6-cyclopropyl-1H-pyrazolo[3,4-b]pyridin-4-yl)-phenyl]-3-(2-fluoro-phenyl)-urea

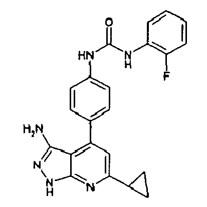

In analogy to the procedure described for example 3a reaction of 175 mg 15a with 62 µl hydrazine hydrate in propanol yielded 35 mg product.
¹H-NMR (d6-DMSO): δ = 0,94 (4H); 2,15 (1 H); 4,45 (2H); 6,80 (1 H); 6,99 (1 H); 7,10 (1 H); 7,20 (1 H); 7,50 (2H); 7,59 (2H); 8,12 (1 H); 8,60 (1 H); 9,26 (1 H); 11,95 (1H) ppm.

### Biologic experiment 1: ELISA - Assay

To prove the high potency activity as inhibitors of Tie2 kinase and Tie2 autophosphorylation the following ELISA-method was established and used.

Herein CHO cell-cultures, which are stably transfected by known techniques with Tie2 using DHFR deficiency as selection marker, are stimulated by angiopoietin-2. The specific autophosphorylation of Tie2 receptors is quantified with a sandwich-ELISA using anti-Tie2 antibodies for catch and anti-phosphotyrosine antibodies coupled to HRP as detection.

### Materials:

96well tissue culture plate, sterile, Greiner
96well FluoroNunc plate MaxiSorp Surface C, Nunc
96well plate polypropylene for compound dilution in DMSO
CHO Tie2/DHFR (transfected cells)
PBS-; PBS++, DMSO
MEM alpha Medium with Glutamax-I without Ribonucleosides and Deoxyribonucleosides (Gibco #32561-029) with 10% FCS after dialysis! and 1% PenStrep

| | |
|---|---|
| Lysis buffer: | 1 Tablet "Complete" protease inhibitor 1 cap Vanadate (1 mL > 40 mg/mL; working solution 2 mM) ad 50 mL with Duschl-Puffer pH 7.6 |
| Anti-TIE-II antibody 1 : 425 in Coating Buffer pH 9.6 | |
| Stock solution: 1.275 mg/mL > working.: 3 µg/mL | |
| PBST:2 bottles PBS(10x) + 10ml Tween, fill up with VE-water | |
| RotiBlock 1 : 10 in VE-water | |
| Anti-Phosphotyrosine HRP-Conjugated 1 : 10000 in 3% TopBlock | |
| | 3% TopBlock in PBST |
| BM Chemiluminescence ELISA Substrate (POD) | |
| | solution B 1 : 100 solution A |
| SF9 cell culture medium | |
| Ang2-Fc in SF9 cell culture medium | |

### Cell experiment:

Dispense 5 x 10⁴ cells / well / 98 µL in 96well tissue culture plate
Incubate at 37 °C / 5% CO₂
After 24 h add compounds according to desired concentrations
Add also to control and stimulated values without compounds 2 µL DMSO And mix for a few min at room temperature
Add 100 µL Ang2-Fc to all wells except control, which receives insect medium Incubate 20 min at 37 °C.
Wash 3x with PBS++
Add 100 µl Lysis buffer /well and shake a couple of min at room temperature Store lysates at 20 °C before utilizing for the ELISA

### Performance of sandwich-ELISA

Coat 96well FluoroNunc Plate MaxiSorp Surface C with anti-Tie2 Mab
1 : 425 in Coating buffer pH 9.6; 100 µL / well overnight at 4 °C
Wash 2x with PBST
Blcock plates with 250 µL / well RotiBlock 1 : 10 in VE-water
Incubate for 2 h at room temperature or overnight at 4 °C shaking
Wash 2x in PBST
Add thawed lysates to wells and incubate overnight shaking at 4 °C
Wash 2x with PBST
Add 100 µL/well anti-Phosphotyrosine HRP-Conjugated 1 : 10000 in 3% TopBlock (3% TopBlock in PBST) and incubate overnight under shaking Wash 6x with PBST
Add 100 µL/well BM Chemiluminescence ELISA Substrate (POD solutions 1 und 2 (1 : 100)
Determine luminescence with the LumiCount.

### Biological experiment 2: Tie-2-Kinase HTRF-Assay

To prove the effectiveness of the compound according to the present invention a Tie-2-Kinase HTRF-Assay was established.

Tie-2 phosphorylates tyrosine residues of the artificial substrate polyGAT (biotinylated polyGluAlaTyr). Detection of phosphorylated product is achieved specifically by a trimeric detection complex consisting of the phosphorylated substrate, streptavidin-XLent (SA-XLent) which binds to biotin, and Europium Cryptate-labeled anti-phosphotyrosine antibody PT66 which binds to phosphorylated tyrosine. Excitation of Europium fluorescence with 337 nm light results in emission of long-lived light with 620 nm. In case a trimeric detection complex has formed, part of the energy will be transferred to the SA-XLent fluorophore that itself then emits long-lived light of 665 nm (FRET: fluorescence resonance energy transfer). Unphosphorylated substrate does not give rise to light emission at 665nm, because no FRET-competent trimeric detection complex can be formed. Measurement is performed in a Packard Discovery or BMG Rubystar instrument. A-counts (emission at 665 nm) will be divided by B-counts (emission at 620 nm) and multiplicated with a factor of 10000. The resulting numbers are called the "well ratio" of the sample.

### Material:

Enzyme: Tie-2-Kinase, in house, aliquots (12 x 10 mL) stored at -80 °C Substrate: PolyGAT labeled with Biotin (1000 µg / mL); CIS Bio ; # 61GATBLB; aliquots stored at -20 °C
ATP: Amersham Pharmacia Biotech Inc. # 27-2056-01; 100 mM; stored at -20 °C
Antibody: PT66-Eu Cryptate ; CIS Bio ; # 61T66KLB ; 30µg/mL; aliquots stored at -20 °C
SA-XLent ; CIS Bio; # 611 SAXLB ; 1000 µg/mL; aliquots stored at -80 °C Microplates : 384 Well black, SV, Greiner, # 784076

### Solutions:

### Assay buffer:

50 mM HEPES (pH 7.0), 25 mM MgCl₂, 5 mM MnCl₂, 1 mM DTT, 0.5 mM Na₃VO₄, 0.01% (v/v) NP40, 1 x Complete EDTA free

### Enzyme working solution:

Tie-2 stock solution is diluted 1:250 in assay buffer

### Substrate working solution:

PolyGAT (1000 µg/mL; 36.23 µM) is diluted 1:90.6 to 400 nM or 77.3 ng/well, ATP (100 mM) is diluted 1 : 5000 to 20.0 µM. Both dilutions in assay buffer. Final assay concentrations: poly-GAT: 200 nM or 5.25 µg/mL, ATP: 10 µM (1 x Km each).

Detection solution: 50 mM HEPES (pH 7.0), BSA 0.2%, 0.6 M KF, 200 mM EDTA, PT66-Europium Cryptate 2.5 ng/well, SA-XLent Cis Bio 90 ng/well.

### Assay steps

All steps at 20 °C
1. 0.75 µL of compound solution in 30 % (v/v) DMSO
2. add 7 µL of substrate working solution
3. add 7 µL of enzyme working solution
4. incubate 75 min (reaction volume: 14.75 µL)
5. add 8 µL of detection solution
6. incubate 180 min or over night at 4 °C (total volume: 22.75 µL)
7. measure HTRF in Packard Discovery or BMG Rubystar instrument (delay 50 µs, integrated time 400 µs)

Final concentrations (in 14.75 µL reaction volume):
Enzyme: unknown
polyGAT (1 x Km): 200 nM (77.3 ng)
ATP (1 x Km): 10 µM
DMSO: 1.5% (v/v)
Buffer conditions: 50 mM HEPES (pH 7.0), 25 mM MgCl₂, 5 mM MnCl₂, 1 mM DTT, 0.5 mM NaV04, 0.01 % (v/v) NP40, 1 x Complete

### Controls:

C₀: uninhibited reaction (DMSO only)
Cᵢ: inhibited reaction with 20 µM Staurosporine

The compounds presented in this application, in particular Example 1, 2, 3, 9, 12, 14 and 15, have high potency activity as inhibitors of Tie2 kinase and/or Tie2 autophosphorylation as measured with the ELISA-method. The lC₅₀ values are below 5 µM.

## Claims

1. A compound of general formula (1): 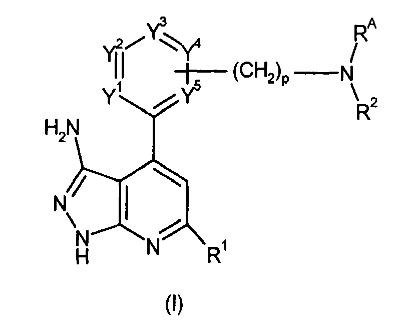 wherein
R¹ stands for C₁-C₆-alkyl, C₂-C₆-alkenyl, C₂-C₆-alkinyl, C₃-C₁₀₋cycloalkyl, C₃-C₁₀-heterocycloalkyl, aryl or heteroaryl optionally being substituted one or more times, the same way or differently with K, whereby C₃-C₁₀-heterocycloalkyl itself must at least once be interrupted by an atom of the group comprising nitrogen, oxygen and/or sulfur and whereby C₃-C₁₀-cycloalkyl ring and/or C₃-C₁₀₋heterocycloalkyl ring can optionally be interrupted one or more times, the same or differently with a group -(CO)-, -SO- or -SO₂- and whereby C₃-C₁₀-cycloalkyl ring and/or C₃-C₁₀-heterocycloalkyl ring can optionally contain one or more double bonds,
K stands for halogen, hydroxy or a substituent of the group comprising -O-R³ or -NR⁵R⁶
or for C₃-C₁₀-cycloalkyl, C₃-C₁₀-heterocycloalkyl, aryl or heteroaryl optionally being substituted one or more times, the same way or differently with L, whereby C₃-C₁₀-heterocycloalkyl itself must at least one time be interrupted by an atom of the group comprising nitrogen, oxygen and/or sulfur and whereby C₃-C₁₀-cycloalkyl ring and/or C₃-C₁₀-heterocycloalkyl ring can optionally be interrupted one or more times, the same or differently with a group -(CO)-, -SO- or -SO₂- and whereby C₃-C₁₀-cycloalkyl ring and/or C₃-C₁₀- heterocycloalkyl ring can optionally contain one or more double bonds,
L stands for a substituent of the group comprising -COR⁴ or -NR⁵R⁶ or for C₁-C₆-alkyl optionally being substituted one or more times, the same way or differently with halogen, hydroxy, cyano, nitro or the group -NR⁵R⁶ or -COR⁴,
R^{A} stands for hydrogen or C₁-C₆-alkyl optionally being substituted one or more times, the same way or differently with halogen, hydroxy, cyano, nitro or the group - NR⁵R⁶ or -COR⁴,
R² stands for a substituent of the group comprising -C(O)-NR⁷R^{7a}, -S(O)₂-R⁷, -S(O)₂NR⁷R^{7a}, -S(O)(NH)R⁷, -C(O)R⁷ or -C(O)OR⁷ ,
R³ stands for C₁-C₆-alkyl, aryl or -(CH₂),-aryl, wherein C₁-C₆-alkyl, aryl or -(CH₂)ₙ-aryl can optionally be substituted one or more times, the same way or differently with C₁-C₆-alkyl, C₁-C₆-alkoxy, halogen, hydroxy, cyano, nitro or the group - NR⁵R⁶ or -COR⁴,
R⁴ stands for hydrogen, hydroxy, C₁-C₆-alkyl, C₁-C₆-alkoxy or the group -NR⁵R⁶,
R⁵ and R⁶ independently from another stand for hydrogen, C₁-C₆-alkyl, aryl or for a group -COR⁴ , wherein C₁-C₆-alkyl or aryl can optionally be substituted one or more times, the same way or differently with halogen, hydroxy, cyano, nitro or the group - NR⁷R^{7a} or -COR⁴,
or
R⁵ and R⁶ together with nitrogen form a 3 to 10 membered heterocycloalkyl ring, whereby heterocycloalkyl ring itself must at least one time be interrupted by an atom of the group comprising nitrogen, oxygen and/or sulfur and can optionally be interrupted one or more times, the same way or differently with a group -(CO)-, -SO- and/or -SO₂- and can optionally contain one or more double bonds,
R⁷ and R^{7a} independently from another stand for hydrogen, C₁-C₆-alkyl, C₃-C₁₀₋cycloalkyl, C₃-C₁₀-heterocycloalkyl, aryl or heteroaryl, wherein C₁₋C₆-alkyl , C₃-C₁₀-cycloalkyl, C₃-C₁₀-heterocycloalkyl, aryl or heteroaryl can optionally be substituted one or more times, the same way or differently with M, whereby C₃-C₁₀-heterocycloalkyl itself must at least one time be interrupted by an atom of the group comprising nitrogen, oxygen and/or sulfur and whereby C₃-C₁₀₋cycloalkylring and/or C₃-C₁₀-heterocycloalkyl ring can optionally be interrupted one or more times, the same way or differently with a group -(CO)-, -SO- or -SO₂- and whereby C₃-C₁₀-cycloalkyl ring and/or C₃-C₁₀-heterocycloalkyl ring can optionally contain one or more double bonds,
or
R⁷ and R^{7a} together with nitrogen form a 3 to 10 membered heterocycloalkyl ring, whereby heterocycloalkyl ring itself must at least one time be interrupted by an atom of the group comprising nitrogen, oxygen and/or sulfur and can optionally be interrupted one or more times, the same way or differently with a group -(CO)-, -SO- or -SO₂- and can optionally contain one or more double bonds,
M stands for a substituent of the group comprising cyano, halogen, hydroxy, nitro or for C₁-C₆-alkyl, C₂-C₆-alkenyl, C₂-C₆-alkinyl, C₁-C₆₋alkoxy, aryl, C₃-C₁₀-heteroaryl, -(CH₂)ₙ-phenyl or -(CH₂)p-O-(CH₂)ₚphenyl, wherein C₁-C₆-alkyl, C₂-C₆-alkenyl, C₂-C₆-alkinyl, C₁₋C₆-alkoxy, aryl, C₃-C₁₀-heteroaryl, -(CH₂)ₙ-pheny) or -(CH₂)p-O-(CH₂)ₚphenyl can optionally be substituted one or more times, the same way or differently with a substituent of the group comprising amino, cyano, halogen, hydroxy, nitro, C₁-C₆-alkoxy, -(CH₂)ₚ-phenyl or -(CH₂)ₚ-O-(CH₂)ₚphenyl,
Y¹, Y², Y³, Y⁴ and Y⁵ independently from another stand for -CH=, -CZ= or -N= and -N= can stand from 0 to 3 times as a ringatom,
Z stands for cyano, nitro, halogen, hydroxy or a substituent of the group comprising -NR⁵R⁶, -OR³, -C(O)R⁷, -C(O)OR⁷, -C(O)NR⁷R^{7a} , -S(O)R⁷ or -S(O)₂R⁷ or for C₁-C₆-alkyl, C₂-C₆-alkenyl, C₂-C₆-alkinyl or C₃-C₁₀-cycloalkyl, whereby C₁-C₆-alkyl, C₂-C₆-alkenyl, C₂-C₆₋alkinyl or C₃-C₁₀-cycloalkyl can optionally be substituted one or more times, the same way or different with cyano, nitro, halogen, hydroxy, -OR³ or -NR⁵R⁶,
n stands for 1 to 4 and
p stands for 0 to 4 as well as
N-oxides, solvates, hydrates, isomers, diastereomers, enantiomers and salts thereof.

2. Compounds of general formula (I) according to claim 1, wherein
R¹ stands for C₁-C₆-alkyl, C₂-C₆-alkenyl, C₂-C₆-alkinyl, C₃-C₁₀₋cycloalkyl, aryl or heteroaryl optionally being substituted one or more times, the same way or differently with K,
K stands for halogen, hydroxy or stands for morpholinyl, piperazinyl, piperidinyl, pyrrolidinyl or phenoxy optionally substituted with L,
L stands for C₁-C₆-alkyl or -COO-C₁-C₆ alkyl, whereby C₁-C₆-alkyl or -COO-C₁-C₆ alkyl can optionally be substituted one or more times, the same way or differently with halogen,
R^{A} stands for hydrogen or C₁-C₆-alkyl optionally being substituted one or more times, the same way or differently with halogen, hydroxy, cyano, nitro or the group - NR⁵R⁶ or -COR⁴,
R² stands for a substituent of the group comprising -C(O)-NR⁷R^{7a}, -S(O)₂-R⁷, -S(O)₂NR⁷R^{7a}, -S(O)(NH)R⁷, -C(O)R⁷ or -C(O)OR⁷,
R³ stands for C₁-C₆-alkyl, aryl or -(CH₂)ₙ-aryl, wherein C₁-C₆-alkyl, aryl or -(CH₂)ₙ-aryl can optionally be substituted one or more times, the same way or differently with C₁-C₆-alkyl, C₁-C₆-alkoxy, halogen, hydroxy, cyano, nitro or the group - NR⁵R⁶ or -COR⁴,
R⁵ and R⁶ independently from another stand for hydrogen, C₁-C₆-alkyl, aryl or for a group -COR⁴, wherein C₁-C₆-alkyl or aryl can optionally be substituted one or more times, the same way or differently with halogen, hydroxy, cyano, nitro or the group - NR⁷R^{7a} or -COR⁴,
R⁷ and R^{7a} independently from another stand for hydrogen, aryl or heteroaryl optionally being substituted one or more times, the same way or differently with M,
M stands for a substituent of the group comprising cyano, halogen, hydroxy, nitro or for C₁-C₆-alkyl, C₂-C₆-alkenyl, C₂-C₆-alkinyl, C₁-C₆₋alkoxy, aryl, C₃-C₁₀-heteroaryl, -(CH₂)ₙ-phenyl or -(CH₂)ₚ-O- (CH₂)pphenyl, wherein C₁-C₆-alkyl, C₂-C₆-alkenyl, C₂-C₆-alkinyl, C₁₋C₆-alkoxy, aryl, C₃-C₁₀-heteroaryl, -(CH₂)ₙ-phenyl or -(CH₂)_{P}-O-(CH₂)ₚphenyl can optionally be substituted one or more times, the same way or differently with a substituent of the group comprising amino, cyano, halogen, hydroxy, nitro or C₁-C₆-alkoxy,
Y¹, Y², Y³, Y⁴ and Y⁵ independently from another stand for -CH=, -CZ= or -N= and -N= can stand from 0 to 3 times as a ringatom,
Z stands for cyano, nitro, halogen, hydroxy or a substituent of the group comprising -NR⁵R⁶, ,-OR³ ,-C(O)R⁷, -C(O)OR⁷, -C(O)NR⁷R^{7a} , -S(O)R⁷ or -S(O)₂R⁷ or for C₁-C₆-alkyl, C₂-C₆-alkenyl, C₂-C₆-alkinyl or C₃-C₁₀-cycloalkyl, whereby C₁-C₆-alkyl, C₂-C₆-alkenyl, C₂-C₆₋alkinyl or C₃-C₁₀-cycloalkyl can optionally be substituted one or more times, the same way or different with cyano, nitro, halogen, hydroxy, -OR³ or -NR⁵R⁶,
n stands for 1 to 4 and
p stands for 0 to 4 as well as
N-oxides, solvates, hydrates, isomers, diastereomers, enantiomers and salts thereof.

3. Compounds of general formula (I) according to claim 1 or 2, wherein
R¹ stands for C₁-C₆-alkyl, C₃-C₁₀-cycloalkyl optionally being substituted one or more times the same way or differently with K,
K stands for halogen, hydroxy or stands for morpholinylene, piperazinylene, piperidinylene or phenyleneoxy,
R^{A} stands for hydrogen or C₁-C₆-alkyl,
R² stands for a substituent of the group comprising -C(O)-NH-R⁷ or -S(O)₂-R⁷,
R⁷ stands for phenylene or naphtylene optionally being substituted one or more times, the same way or differently with M,
M stands for a substituent of the group comprising cyano, halogen, nitro or for C₁-C₆-alkyl optionally being substituted one or more times, the same way or differently with a substituent of the group comprising amino, cyano, halogen, hydroxy, nitro or C₁-C₆-alkoxy,
Y¹, Y², Y³ Y⁴ and Y⁵ stand for -CH= and
p stands for 0 to 4
as well as N-oxides, solvates, hydrates, isomers, diastereomers, enantiomers and salts thereof.

4. Compounds of general formula (I) according to any one of claims 1 to 3, wherein
R¹ stands for tert.-butyl or cyclopropyl,
R^{A} stands for hydrogen,
R² stands for a substituent of the group comprising -C(O)-NH-R⁷ or -S(O)₂-R⁷,
R⁷ stands for phenylene or naphtylene optionally being substituted one or more times, the same way or differently with M,
M stands for a substituent of the group comprising cyano, halogen, nitro or for methyl,
Y¹, Y², Y³ Y⁴ and Y⁵ stand for -CH= and
p stands for 0
as well as N-oxides, solvates, hydrates, isomers, diastereomers, enantiomers and salts thereof.

5. A method of preparing the compound of general formula (I) according to any one of claims 1 to 4, the method comprising the following method steps:
A) an aldehyde of formula 1 is reacted with methylketone of formula 2, an alkyl cyanoacetate and an ammonium acetate into a compound of formula 3 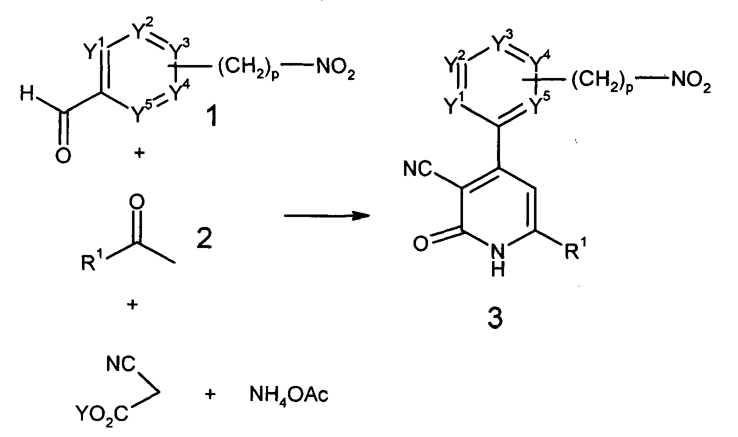
B) a compound of formula 3 reacted to a compound of formula 4 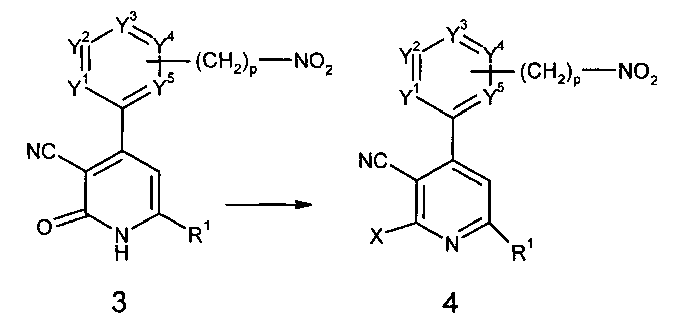
C) a compound of formula 4 reduced to a compound of formula 5 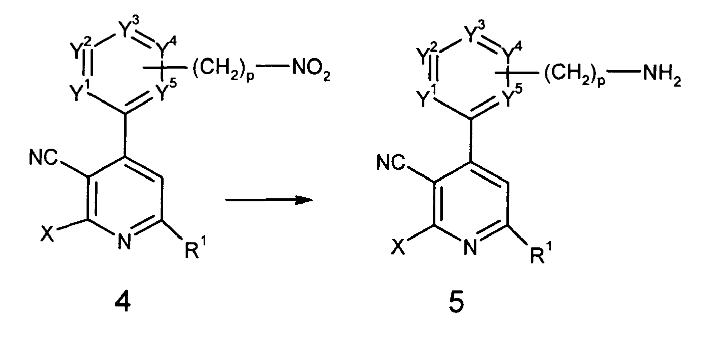
D) a compound of formula 5 reacted to a compound of formula 6 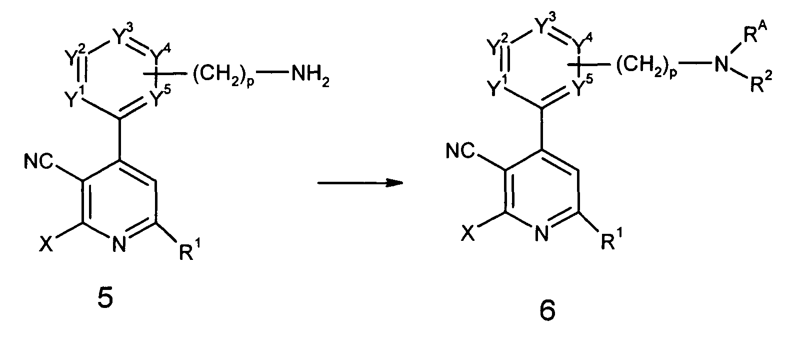
E) a compound of formula 6 reacted by addition of hydrazines to a compound of general formula (I) 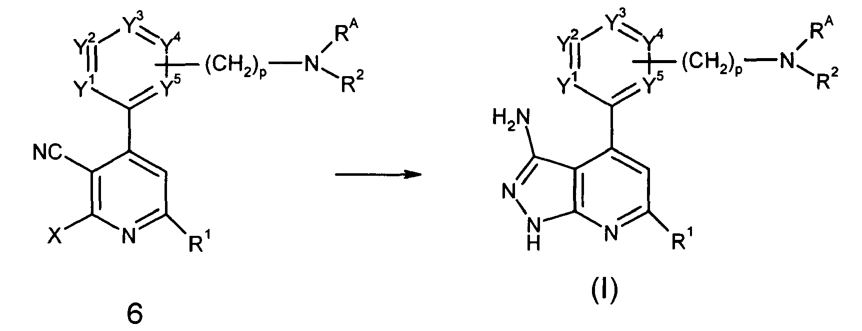
wherein Y stands for C₁-C₄-alkyl, X stands for halogen or perfluor-C₁-C₄-alkyl sulfonyl and R^{A}, R¹, R², Y¹, Y², Y³, Y⁴, Y⁵ and p have the meaning described in general formula (I) according to any one of claims 1 to 4 as well as N-oxides, solvates, hydrates, isomers, diastereomers, enantiomers and salts thereof.

6. A method for preparing a compound of general formula (I) according to any one of claims 1 to 4, the method comprising the following method steps:
A) a compound of formula 7 reacted to a compound to formula 8 and 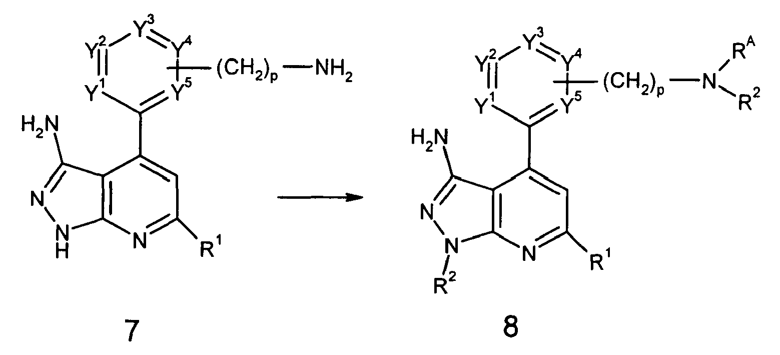
B) a compound of formula 8 reacted to a compound of general formula (I) 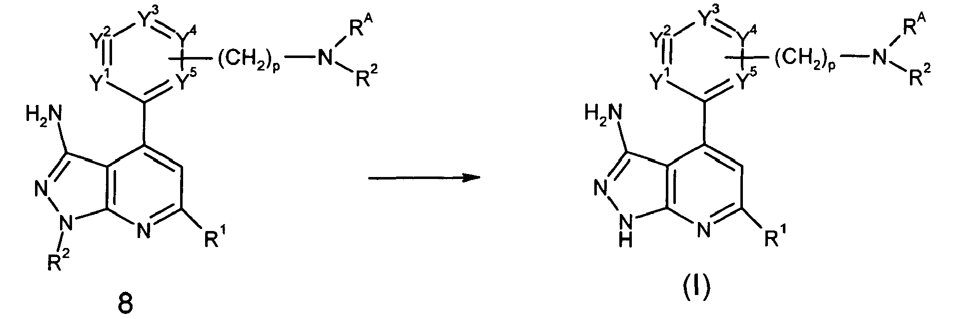
wherein R^{A}, R¹, R², Y¹, Y², Y³, Y⁴, Y⁵ and p have the meaning described in general formula (I) according to any one of claims 1 to 4 as well as N-oxides, solvates, hydrates, isomers, diastereomers, enantiomers and salts thereof.

7. A pharmaceutical composition which comprises the compounds of general formula (I) according to any one of claims 1 to 4 or a pharmaceutically acceptable salt or an in vivo hydrolysable ester thereof, and a pharmaceutically-acceptable diluent or carrier.

8. A use of the compound of any one of claims 1 to 4 for manufacturing a pharmaceutical composition for the treatment of diseases of dysregulated vascular growth or of diseases which are accompanied with dysregulated vascular growth.

9. The use according to claim 8, wherein the diseases are tumors and / or metastases thereof.

10. The use according to claim 8, wherein the diseases are retinopathy, other angiogenesis dependent diseases of the eye, rheumatoid arthritis, and other inflammatory diseases associated with angiogenesis.

11. The use of claim 10, wherein the angiogenesis dependent diseases of the eye are cornea transplant rejection, age-related macular degeneration.

12. The use according to claim 11, wherein the diseases are coronary and peripheral artery disease.

13. The use of claim 10, wherein the inflammatory diseases associated with angiogenesis are psoriasis, delayed type hypersensitivity, contact dermatitis, asthma, multiple sclerosis, restenosis, pulmonary hypertension, stroke, and diseases of the bowel.

14. The use according to claim 8, wherein the diseases are ascites, edema like brain tumour associated edema, high altitude trauma, hypoxia induced cerebral edema pulmonary edema and macular edema or edema following burns and trauma, chronic lung disease, adult respiratory distress syndrome, bone resorbtion and for benign proliferating diseases like myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.

15. A method for treating a disease of dysregulated vascular growth or diseases which are accompanied with dysregulated vascular growth administering an effective amount of a compound of general formula (I) according to any one of claims 1 to 4.

16. The method according to claim 15, wherein the disease is tumor and / or metastases thereof.

17. The method according to claim 15, wherein the diseases are retinopathy, other angiogenesis dependent diseases of the eye rheumatoid arthritis, and other inflammatory diseases associated with angiogenesis.

18. The method according to claim 17, wherein the angiogenesis dependent diseases of the eye are cornea transplant rejection, age-related macular degeneration.

19. The method according to claim 18, wherein the diseases are coronary and peripheral artery disease.

20. The method according to 17, wherein the inflammatory diseases associated with angiogenesis are psoriasis, delayed type hypersensitivity, contact dermatitis, asthma, multiple sclerosis, restenosis, pulmonary hypertension, stroke, and diseases of the bowel.

21. The method according to claim 15, wherein the diseases are ascites, edema like brain tumour associated edema, high altitude trauma, hypoxia induced cerebral edema pulmonary edema and macular edema or edema following burns and trauma, chronic lung disease, adult respiratory distress syndrome, bone resorbtion and for benign proliferating diseases like myoma, benign prostate hyperplasia and wound healing for the reduction of scar formation, reduction of scar formation scar formation during regeneration of damaged nerves, endometriosis, pre-eclampsia, postmenopausal bleeding and ovarian hyperstimulation.
